# FASCICULE DE BREVET EUROPEEN

(11) **EP 1 558 203 B1**
(45) Date de publication et mention de la délivrance du brevet: **09.05.2007**
(21) Numéro de dépôt: 03778455.0
(22) Date de dépôt: 24.10.2003
(51) Int. Cl.: A61K 8/49, A61Q 5/00, A61Q 7/00

(54) **COMPOSITION CAPILLAIRE CONTENANT UN COMPOSE STYRYL-PYRAZOLE**
HAARPFLEGEMITTEL ENTHALTEND EINE STYRYLPYRAZOLE VERBINDUNG
HAIR TREATMENT COMPOSITION CONTAINING A STYRYL-PYRAZOLE COMPOUND

(30) Priorité: 29.10.2002 FR 0213522; 12.11.2002 US 425276 P
(43) Date de publication de la demande: 03.08.2005
(73) Titulaire: L'ORÉAL, 75008 Paris (FR)
(72) Inventeur: BOULLE, Christophe, F-75005 Paris (FR); ROZOT, Roger, F-77400 Lagny/Marne (FR)
(74) Mandataire: Miszputen, Laurent
(86) Numéro de dépôt international: PCT/FR2003/003160
(87) Numéro de publication internationale: WO 2004/039306

(56) Documents cités:
- EP-A- 0 245 825
- US-A- 4 924 002
- US-B1- 6 331 555
- DATABASE CHEMCATS [Online] XP002278040 extrait de STN Database accession no. 2002:1799670
- DATABASE CHEMCATS [Online] XP002278041 extrait de STN Database accession no. 2002:1966519
- DATABASE CHEMCATS [Online] XP002278042 extrait de STN Database accession no. 2002:1799727
- DATABASE CHEMCATS [Online] XP002278043 extrait de STN Database accession no. 2003:1939884
- DATABASE CHEMCATS [Online] XP002278044 extrait de STN Database accession no. 2002:1954990
- DATABASE CHEMCATS [Online] XP002278045 extrait de STN Database accession no. 2002:1942261

## Description

### DOMAINE DE L'INVENTION

L'invention a pour objet une composition de soin capillaire contenant une quantité efficace d'un composé pyrazolique et plus spécialement d'un styryl-pyrazole, destinée à induire et/ou stimuler la croissance des fibres kératiniques notamment humaines et/ou freiner leur chute. Elle se rapporte, en outre, à un procédé de traitement cosmétique ainsi qu'à de nouveaux composés styryl-pyrazole, destinés à stimuler la croissance des fibres kératiniques et/ou freiner leur chute.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

En particulier, l'invention a trait à une composition de soin ou de maquillage des cheveux ou des cils, contenant une quantité efficace d'un composé styryl-pyrazole, destinée à augmenter leur densité et/ou améliorer leur aspect.

### ARRIERE PLAN DE L'INVENTION

La croissance des cheveux et leur renouvellement sont principalement déterminés par l'activité des follicules pileux et de leur environnement matriciel. Leur activité est cyclique et comporte essentiellement trois phases à savoir la phase anagène, la phase catagène et la phase télogène.

A la phase anagène (phase active ou de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10% environ sont au repos et seront remplacés en quelques mois.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

En outre, différentes causes peuvent entraîner une perte importante, temporaire ou définitive, des cheveux. Il peut s'agir de chute et d'altération des cheveux au décours d'une grossesse (post partum), au cours d'états de dénutrition ou de déséquilibres alimentaires ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Les cycles de croissance successifs aboutissent à des cheveux de plus en plus fins et de plus en plus courts, se transformant peu à peu en un duvet non pigmenté, entraînant ainsi un appauvrissement progressif de la chevelure. Des zones sont touchées préférentiellement, notamment les golfes temporaux ou frontaux chez l'homme, et chez les femmes, on constate une alopécie diffuse du vertex.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique, la malnutrition, peuvent accentuer le phénomène.

Dans certaines dermatoses du cuir chevelu à caractéristique inflammatoire, telles que par exemple le psoriasis ou les dermatites séborrhéïques, la chute des cheveux peut être fortement accentuée ou entraîner des cycles des follicules fortement perturbés.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute.

Dans cette optique, on a déjà proposé un grand nombre de compositions comprenant des actifs très divers, comme par exemple le 2,4-diamino 6-pipéridinopyrimidine 3-oxyde ou "minoxidil" décrit dans les brevets US 4 139 619 et US 4 596 812 ou encore ses nombreux dérivés comme ceux décrits par exemple dans les documents EP 0353123, EP 0356271, EP 0408442, EP 0522964, EP 0420707, EP 0459890, EP 0519819.

Des études cliniques ont démontré que des analogues de PGF2-α avaient la propriété de provoquer la croissance de poils et de cils chez l'homme et chez l'animal (Murray A. and Johnstone M.D., 1997. *Am. J. Opht.,* 124(4), 544-547). Chez l'homme, des essais réalisés sur le cuir chevelu ont montré qu'un analogue de prostaglandine E2 (le viprostol) avait la propriété d'augmenter la densité capillaire (Roenigk HH., 1988. *Clinic Dermatol.,* 6(4), 119-121).

Par ailleurs, le document WO 98/33497 décrit des compositions pharmaceutiques contenant des prostaglandines ou des dérivés de prostaglandines, destinées à lutter contre la chute des cheveux chez l'homme. Les prostaglandines du type A2, F2α et E2 sont mentionnées comme préférées.

Cependant, les prostaglandines sont des molécules au temps de demi-vie biologique très court et agissant de façon autocrine ou paracrine, ceci traduisant le caractère local et labile du métabolisme des prostaglandines (Narumiya S. et al., 1999, *Physiol. Rev.,* 79(4), 1193-1226).

Il apparaît donc comme important, pour maintenir et/ou augmenter la densité capillaire chez l'homme de préserver les réserves endogènes de PGF2-α comme de PGE2 des différents compartiments du follicule pileux ou de son environnement cutané proche.

Une solution donnant de bons résultats est l'utilisation de composés inhibiteurs de lipoxygénase et/ou inducteurs de la cyclo-oxygénase en vue de favoriser la croissance des cheveux ; une hypothèse est que l'utilisation de tels composés oriente le métabolisme des acides gras vers la synthèse endogène de prostaglandines de préférence à d'autres voies.

Toutefois, pour améliorer encore les résultats, il serait souhaitable de pouvoir prolonger l'activité des prostaglandines impliquées dans la croissance et le maintien du cheveu en vie.

Il est par ailleurs bien connu que les programmes de différenciation des kératinocytes de l'épiderme et du follicule pileux sont clairement différents. Ainsi, il est connu que les kératines de la tige pilaire représente une famille (Langbein et al., 2001, J. Biol. Chem. 276 : 35123-35132) distincte de celle exprimée dans l'épiderme, que les marqueurs de différenciation tels que les kératines K₁ et K₁₀ ne sont pas exprimés dans le follicule pileux et en particulier dans la gaine externe (Lenoir et al., 1988, Dev. Biol. 130 : 610-620), que la trichohyaline (O'Guin et al., 1992, J. Invest. Dermatol. 98 : 24-32) et la kératine K6irs (Porter et al., 2001, Br. J. Dermatol. 145 : 558-568) sont exprimées dans le follicule pileux en particulier dans la gaine interne mais pas dans l'épiderme, et que la cyclo-oxygénase de type 1, si elle est exprimée dans l'épiderme, ne l'est pas dans les kératinocytes du follicule pileux mais dans la papille dermique (Michelet. et al., 1997, J. Invest. Dermatol. 108 : 205-209).

Le demandeur a maintenant mis en évidence qu'une enzyme spécifiquement impliquée dans la dégradation de ces prostaglandines est présente dans la papille dermique du cheveu, qui est un compartiment déterminant pour la vie du cheveu. En effet, le demandeur a maintenant prouvé la présence de 15-hydroxy prostaglandine déshydrogénase (15-PGDH en abréviation) à ce niveau. II a en outre montré que l'inhibition de la 15-PGDH a un effet bénéfique sur la croissance pilaire.

C'est pourquoi la présente invention se rapporte à une composition de soin ou de traitement des fibres kératiniques humaines et notamment capillaires contenant au moins un inhibiteur particulier de la 15-hydroxy prostaglandine déshydrogénase et un milieu physiologiquement acceptable.

La 15-PGDH est une enzyme clé dans la désactivation des prostaglandines, en particulier de la PGF2-α, et de la PGE2, qui sont des médiateurs importants de la croissance et la survie du cheveu. Elle répond à la classification EC 1.1.1.141 et est NAD+ dépendante. Elle a été isolée de rein de porc ; on a notamment observé son inhibition par une hormone thyroïdienne, la tri-iodo thyronine, à des doses très supérieures aux doses physiologiques.

Cependant, il n'avait jamais été proposé d'utiliser un inhibiteur de 15-PGDH pour maintenir et/ou augmenter la densité des fibres kératiniques humaines et notamment la densité capillaire et/ou pour réduire l'hétérogénéité des diamètres des fibres kératiniques et notamment des cheveux chez l'homme. Par augmenter la densité des fibres kératiniques, et notamment la densité capillaire, on entend augmenter le nombre des fibres kératiniques, et notamment des cheveux par cm² de peau ou de cuir chevelu.

### EXPOSE DE L'INVENTION

Le demandeur a trouvé que certains composés pyrazoliques et notamment certains styryl-pyrazoles salifiés ou non, sont d'une façon surprenante dotés d'une activité favorable à l'amélioration de la densité des fibres kératiniques humaines notamment la densité capillaire. Il a par ailleurs trouvé que ces composés sont des inhibiteurs de la 15-hydroxy prostaglandine déshydrogénase.

La présente invention a donc pour objet l'**utilisation notamment cosmétique d'au moins un composé pyrazolique de formule (I) ou de l'un de ses sels :** 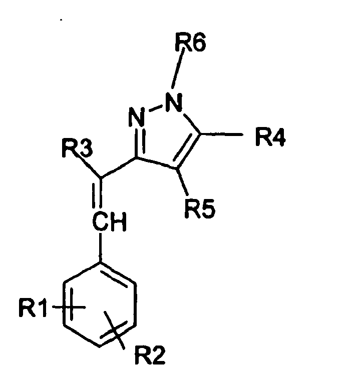 dans laquelle :
- R₁, R₂, R₄ et R₅ identiques ou différents sont choisis parmi l'hydrogène, un halogène, les groupes OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇. les radicaux alkyle, saturés ou insaturés, linéaires ou ramifiés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués par au moins un substituant A₁, avec R₇, R'₇, R"₇ et R"'₇, désignant indépendamment l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₂ ;
- R₃ est choisi parmi CN, COOR₈, CONR₈R'₈, COR₈, SO₂R₈, SO₂NR₈R'₈, avec R₈ et R'₈ désignant indépendamment l'hydrogène, un radical alkyle en C₁-C₂₀, linéaire ou ramifié ou un cycle de 4 à 7 atomes, isolé ou accolé à un autre cycle et contenant éventuellement au moins un hétéroatome, le radical alkyle ou lesdits cycles étant saturés ou non et éventuellement substitués par un au moins un substituant A₃ ;
- R₆ est choisi parmi l'hydrogène, les groupes COOR₉, COR₉, CSR₉, COSR₉, CONR₉R'₉, SO₂R₉, SO₂NR₉R'₉, les radicaux alkyle en C₁-C₂₀, linéaires ou ramifiés, saturés ou non et les cycles de 4 à 7 atomes, saturés ou insaturés, contenant éventuellement au moins un hétéroatome, ces cycles pouvant être séparés ou accolés, les radicaux alkyle et les cycles pouvant, en outre, être, substitués par au moins un substituant A₄, avec R₉ et R'₉ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₅ ;
- A₁, A₂, A₃, A₄ et A₅ étant indépendamment choisis parmi les halogènes, les groupes OR₁₀, SR₁₀; NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R"₁₀, NR₁₀CSNR'₁₀R"₁₀, NR₁₀CSR'₁₀, avec R₁₀, R'₁₀, R"₁₀ et R"'₁₀ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non. comme agent pour induire et/ou stimuler la croissance des fibres kératiniques notamment humaines comme les cils et les cheveux des êtres humains et/ou freiner leur chute et/ou augmenter leur densité.

L'invention se rapporte encore à une composition de soin et/ou de maquillage des fibres kératiniques notamment humaines, et plus spécialement une composition de soin capillaire ou de mascara à application topique contenant dans un milieu physiologiquement acceptable une quantité efficace d'un composé styryl-pyrazole de formule (1) ou d'un de ses sels , caractérisée par le fait que R₁ et R₂ sont situés sur le cycle phénylique en position ortho du branchement de la partie pyrazole.

L'invention se rapporte encore également à une composition de soin et/ou de maquillage des fibres kératiniques notamment humaines, et plus spécialement une composition de soin capillaire ou de mascara à application topique contenant dans un milieu physiologiquement acceptable une quantité efficace d'un composé styryl-pyrazole de formule (III) ou d'un de ses sels que l'on définira plus loin en détail.

L'invention s'applique aussi aux fibres kératiniques des mammifères de l'espèce animale (chien, cheval ou chat par exemple).

L'invention se rapporte encore à l'utilisation cosmétique d'au moins un composé pyrazolique de formule (I) ou d'un de ses sels dans une composition cosmétique de soin et/ou de maquillage des fibres kératiniques humaines pour induire et/ou stimuler leur croissance, freiner leur chute et/ou augmenter leur densité et/ou traiter l'alopécie androgénique ainsi qu'à l'utilisation d'au moins un composé de formule (I) ou de l'un de ses sels pour la préparation d'une composition de soin ou de traitement des fibres kératiniques humaines, destinée à induire et/ou stimuler la croissance des fibres et/ou freiner leur chute et/ou augmenter leur densité et/ou traiter l'alopécie androgénique.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

L'invention se rapporte encore à l'utilisation cosmétique d'au moins un composé pyrazolique de formule (I) ou de l'un de ses sels dans une composition cosmétique de soin capillaire d'être humain pour réduire la chute des cheveux et/ou augmenter leur densité. Elle a encore pour objet l'utilisation d'au moins un composé pyrazolique de formule (1) ou de l'un de ses sels pour la préparation d'une composition capillaire pour être humain, destinée à induire et/ou stimuler la croissance des cheveux et/ou freiner leur chute et/ou augmenter leur densité.

En particulier, l'invention se rapporte à l'utilisation cosmétique d'au moins un composé pyrazolique de formule (I) ou de l'un de ses sels dans une composition cosmétique de soin capillaire d'être humain ou pour la préparation d'une composition capillaire d'être humain pour traiter ou destinée à traiter l'alopécie d'origine naturelle et en particulier androgénique ou andro-chrono-génétique. Ainsi, cette composition permet de maintenir en bon état la chevelure et/ou lutter contre la chute naturelle des cheveux et plus spécialement celles des hommes.

L'invention a encore pour objet l'utilisation cosmétique d'au moins un composé pyrazolique de formule (I) ou d'un de ses sels, dans une composition cosmétique de soin et/ou de maquillage des cils d'être humain, pour induire et/ou stimuler la croissance des cils et/ou augmenter leur densité ainsi que l'utilisation d'au moins un composé de formule (I) ou d'un de ses sels, pour la préparation d'une composition de soin et/ou de traitement des cils d'être humain, destinée à induire et/ou stimuler la croissance des cils et/ou augmenter leur densité. Cette composition permet ainsi de maintenir en bon état les cils et/ou améliorer leur état et/ou leur aspect.

L'invention a également pour objet un procédé de traitement cosmétique des fibres kératiniques (cheveux ou cils notamment) et/ou de la peau d'où émergent lesdites fibres, y compris du cuir chevelu et des paupières, en particulier destiné à stimuler la croissance des fibres kératiniques d'être humain et/ou freiner leur chute, caractérisé par le fait qu'il consiste à appliquer sur les fibres kératiniques et/ou la peau d'où émergent lesdites fibres, une composition cosmétique comprenant une quantité efficace d'au moins un composé de formule (I) ou d'un de ses sels, à laisser celle-ci en contact avec les fibres kératiniques et/ou la peau d'où émergent lesdites fibres, et éventuellement à rincer les fibres et/ou ladite peau.

Ce procédé de traitement présente les caractéristiques d'un procédé cosmétique dans la mesure où il permet d'améliorer l'esthétique des fibres kératiniques en leur donnant une plus grande vigueur et un aspect amélioré. En outre, il peut être utilisé quotidiennement pendant plusieurs mois, sans prescription médicale.

Ainsi, la présente invention a également pour objet un procédé de traitement cosmétique des cheveux et/ou du cuir chevelu, destiné à stimuler la croissance des cheveux d'être humain et/ou freiner leur chute, caractérisé par le fait qu'il consiste à appliquer sur les cheveux et/ou le cuir chevelu, une composition cosmétique comprenant une quantité efficace d'au moins un composé de formule (1) ou d'un de ses sels, à laisser celle-ci en contact avec les cheveux et/ou le cuir chevelu, et éventuellement à rincer les cheveux et/ou le cuir chevelu.

Ce procédé de traitement présente les caractéristiques d'un procédé cosmétique dans la mesure où il permet d'améliorer l'esthétique des cheveux en leur donnant une plus grande vigueur et un aspect amélioré. En outre, il peut être utilisé quotidiennement pendant plusieurs mois.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant une quantité efficace d'au moins un composé de formule (I) ou l'un de ses sels, à laisser celle-ci en contact avec les cheveux et/ou le cuir chevelu, et éventuellement à rincer les cheveux et/ou le cuir chevelu.

L'invention a encore pour objet un procédé de soin cosmétique et/ou de maquillage des cils humains, en vue d'améliorer leur état et/ou leur aspect, caractérisé en ce qu'il consiste à appliquer sur les cils et/ou les paupières une composition de mascara comprenant au moins un composé de formule (I) ou l'un de ses sels et à laisser celle-ci au contact des cils et/ou les paupières. Cette composition de mascara peut être appliquée seule ou en sous-couche d'un mascara pigmenté classique et être éliminée comme un mascara pigmenté classique.

L'invention a encore pour objet une composition de soin ou de maquillage des fibres kératiniques, comprenant dans un milieu physiologiquement acceptable, en particulier cosmétique, au moins un composé de formule (1) ou fun de ses sels et au moins un actif additionnel favorisant la repousse des fibres kératiniques humaines et/ou limitant la chute choisi parmi l'aminexil, les agonistes du récepteur FP et les vasodilatateurs et plus spécialement choisi parmi l'aminexil, le minoxidil, le latanoprost, le butaprost et le travoprost.

L'invention a aussi pour objet l'utilisation cosmétique d'au moins un composé styryl-pyrazole de formule (1) ou d'un de ses sels dans une composition cosmétique comme agent pour préserver la quantité et/ou l'activité des prostaglandines au niveau du follicule pileux.

L'invention a encore pour objet l'utilisation d'au moins un composé styryl-pyrazole de formule (I) ou d'un de ses sels pour la fabrication d'une composition destinée à préserver la quantité et/ou l'activité des prostaglandines au niveau du follicule pileux.

L'invention a encore pour objet l'utilisation d'au moins un composé pyrazolique de formule (I) ou de l'un de ses sels comme inhibiteur de la 15-hydroxy prostaglandine déshydrogénase de la peau humaine. Elle a encore pour objet l'utilisation d'au moins un composé pyrazolique de formule (1) ou de l'un de ses sels pour la fabrication d'une composition destinée à traiter les désordres liés à la 15-hydroxy prostaglandine déshydrogénase en particulier chez l'être humain.

### DESCRIPTION DÉTAILLÉE DES MODES DE RÉALISATION DE L'INVENTION

Par inhibiteur de la 15-hydroxy prostaglandine déshydrogénase, on entend un composé de formule (I) qui soit capable d'inhiber ou de diminuer l'activité de l'enzyme 15-PGDH notamment de l'homme, et/ou capable d'inhiber, diminuer ou ralentir la réaction catalysée par cette enzyme.

Selon un mode de réalisation avantageux de l'invention, le composé de formule (I) est un inhibiteur spécifique de la 15-PGDH ; par inhibiteur spécifique on entend un composé de formule (1) qui soit peu ou pas inhibiteur de la synthèse des prostaglandines, en particulier de ,la synthèse de PGF2-α ou de PGE2. Selon un mode de mise en oeuvre particulier de l'invention, l'inhibiteur de la 15-PGDH est peu ou pas inhibiteur de la synthèse des prostaglandines, en particulier de la synthèse de PGF2-α ou de PGE2. Selon un mode particulier de mise en oeuvre de l'invention, l'inhibiteur de la 15-PGDH n'est peu ou pas inhibiteur de la prostaglandine synthase (PGF synthase).

En effet, le demandeur a maintenant trouvé, que la PGF synthase est également exprimée dans la papille dermique. Le maintien d'une quantité efficace de prostaglandines au site d'action résulte donc d'un équilibre biologique complexe entre la synthèse et la dégradation de ces molécules. L'apport exogène de composés inhibant le catabolisme sera donc moins efficace si cette activité est combinée à une inhibition de la synthèse.

Dans la suite du texte, et sauf mention exprès, l'emploi du terme composé de formule (1) doit être compris comme signifiant aussi bien le composé de formule (I) sous forme non ionique que sous forme de sel.

Avantageusement, les composés de formule (I) présentent une activité inhibitrice de la 15-PGDH qui est supérieure à l'activité d'inhibition de la PGF synthase. En particulier, le rapport entre les activités inhibitrices respectivement de la PGF synthase et de la 15-PGDH pour une concentration donnée, déterminées notamment par les concentrations inhibitrices de 50 % de l'activité enzymatique respectivement de la PGFsynthase, IC_{50fs}, et de la 15-PGDH, IC_{50dh}, est au moins supérieur à 1, et notamment d'au moins 3:1, avantageusement supérieur ou égal à 5:1. Les composés préférés de l'invention présentent un ratio IC_{50fs}/IC_{50dh} supérieur ou égal à 10:1, et en particulier supérieur ou égal à 15.

"Au moins un" selon l'invention signifie un ou plusieurs (2, 3 ou plus). En particulier, la composition peut contenir un ou plusieurs composés de formule (1). Ce ou ces composés peuvent être des isomères cis ou trans ou Z ou E ou un mélange d'isomères cis/trans ou Z/E En particulier, le cycle aromatique peut être en position cis ou trans ou Z ou E et mieux en position Z par rapport au cycle pyrazole. Ce ou ces composés peuvent aussi être sous forme tautomère. Ils peuvent être également des énantiomères et/ou des diastéréoisomères ou un mélange de ces isomères, en particulier un mélange racémique.

Selon l'invention, les cycles employés pour R₁ à R₁₀, R'₇, R"₇, R"'₇, R'₈, R'₉, R'₁₀, R"₁₀ et R"'₁₀ comportent de 4 à 7 atomes et mieux de 5 à 6 atomes. Ils peuvent être saturés ou insaturés et comporter éventuellement un ou plusieurs hétéroatomes tels que S, N, O ou leurs associations. Ils peuvent être seuls ou accolés à un autre cycle qui peut être identique ou différent. Comme cycles carbonés saturés utilisables on peut citer le radical cyclopentyle ou cyclohexyle. Comme hétérocycle, on peut citer les cycles pyridine, pipéridine, morpholine, pyrrole, furanne, thiazole. Comme cycles carbonés insaturés, on peut citer le radical phényle, naphtyle. En outre, ces cycles peuvent être substitués par un substituant ayant la définition indiquée ci-dessus pour A₁. Avantageusement, lorsque R₆ comporte un ou plusieurs hétéroatomes, la liaison avec l'azote du cycle pyrazole se fait sous forme d'une liaison N-C.

Selon un mode de réalisation de l'invention, le ou les substituants portés par les radicaux alkyle ou aryle, à savoir A₁ à A₅ sont les atomes d'halogène et notamment les atomes de chlore, brome, iode ou fluor, de préférence les atomes de chlore ou les radicaux alkyle en C₁-C₂₀, linéaires ou ramifiés ou encore les radicaux alkyle perfluorés. Comme exemple de radicaux alkyle perfluorés utilisables, on peut citer CF₃.

Par "radical alkyle" on entend au sens de l'invention un radical hydrocarboné qui peut être linéaire ou ramifié et saturé ou insaturé. De préférence, le radical alkyle comporte de 1 à 10 atomes de carbone.

Comme exemple de radical alkyle utilisable selon l'invention, on peut citer méthyle, éthyle, iso-propyle, n-butyle, n-hexyle, éthyl-2-héxyle, éthylène, propylène.

Selon l'invention, les composés de formule (1) (ou leur(s) sel(s)) sont sous forme isolée, c'est-à-dire non polymérique. En outre, R₁ et R₂ peuvent être situés en toute position du cycle phénylique et en particulier en position ortho du branchement de la partie pyrazole.

De préférence, l'un au moins des R₁ et R₂ représentent un atome d'hydrogène OR₇ CF₃, un atome d'halogène et notamment un atome de chlore, -R₇ représentant un radical alkyle en C₁-C₁₀ et par exemple méthyle. En particulier, R₁ et/ou R₂ représentent un atome d'halogène et notamment de chlore.

Avantageusement, R₃ représente CN, COOR₈, CONR₈R'₈, COR₈ et par exemple CN.

Selon un mode de réalisation de l'invention, R₄, R₅, R₆ représentent indépendamment l'un de l'autre un radical alkyle en C₁-C₁₀ éventuellement substitué par OR₁₀ comme CH₂CH₂OR₁₀, NH₂, H, CN, ou un cycle hydrocarboné, saturé ou non, comme un cycle phényle, R₁₀ représentant par exemple H. Avantageusement, R₆ représente CH₂CH₂OR₁₀ et en particulier CH₂CH₂OH ou le radical phényle. De préférence, R₄ représente NH₂ ou H. Selon un mode avantageux, R₅ représente CN ou H.

Par sels de composé de formule (1) on entend selon l'invention, les sels organiques ou inorganiques d'un composé de formule (I).

Comme sels inorganiques utilisables selon l'invention on peut citer les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), d'ammonium et de manganèse (Mn²⁺) ; les hydroxydes, les carbonates, les halogénures (chlorures), les sulfates, les nitrates, les phosphates.

Les sels organiques utilisables selon l'invention sont par exemple les sels de tri-éthanolamine, mono-éthanolamine, di-éthanolamine, hexadécylamine, N,N,N',N'-tétrakis-(hydroxy-propyl-2) éthylène diamine, tris-hydroxyméthyl aminométhane.

Selon un mode particulier de réalisation de l'invention, les composé pyrazoliques auxquels s'applique l'invention présentent la formule (II) ou un de ses sels : 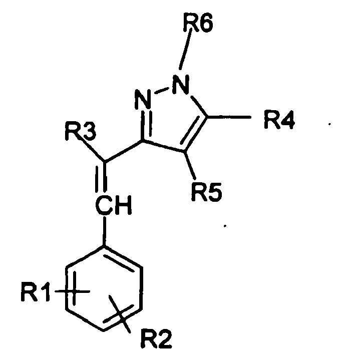 dans laquelle:
- R₁, R₂, R₄ et R₅ représentent indépendamment H, un halogène, OR₇, SR₇, NR₇R'₇, COOR₇, CONR₇R'₇, CF₃, CN, un radical alkyle, saturé ou non, en C₁-C₁₀, un cycle saturé ou non, séparé ou accolé à un autre cycle, contenant éventuellement au moins un hétéroatome, les radicaux alkyle et les cycles pouvant, en outre, être substitués par au moins un substituant A₁, avec R₇, R₇' désignant indépendamment H, un radical alkyle en C₁-C₁₀ ou un cycle isolé ou accolé à un autre cycle ;
- R₃ représente CN, COOR₈, CONR₈R'₈, COR₈, avec R₈ et R'₈ désignant indépendamment H, un radical alkyle en C₁-C₁₀ ou un cycle isolé ou accolé à un autre cycle et contenant éventuellement au moins un hétéroatome, lesdits cycles étant saturés ou non et éventuellement substitués par au moins un substituant A₁;
- R₆ représente l'hydrogène, COOR₉, COR₉, un radical alkyle en C₁-C₁₀ saturé ou non ou un cycle saturé ou non, séparé ou accolé à un autre cycle, contenant éventuellement au moins un hétéroatome, les radicaux alkyle et les cycles pouvant, en outre, être, substitués par au moins un substituant A₁, avec R₉ et R'₉ désignant indépendamment H, un radical alkyle en C₁-C₂₀ ou un cycle isolé ou accolé à un autre cycle ;
- les cycles ayant de 5 à 6 atomes ;
- les hétéroatomes étant O, N, S ou leur association.

Avantageusement, le composé de formule (I) ou (II) est sous forme Z.

Selon un autre mode de réalisation de l'invention, les composés pyrazoliques présentent la formule (III) suivante ou un de ses sels: 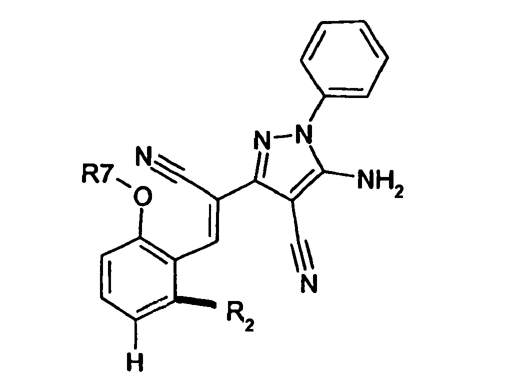 R₇ représente
a) un radical alkyle en C₁-C₁₀, linéaire ou ramifié, saturé ou non, éventuellement substitué par au moins un substituant A₁ ; ou
b) un cycle C¹ de 4 à 7 atomes, saturé ou insaturé, contenant éventuellement au moins un hétéroatome et/ou étant éventuellement substitué par au moins un substituant A₁ et/ou éventuellement accolé à au moins un cycle C² de 4 à 7 atomes, saturé ou insaturé, contenant éventuellement au moins un hétéroatome ;
R₂ représente
. OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R'"₇. NR₇CSR'₇, NR₇CSNR'₇R"₇, un radical alkyle, saturé ou non, en C₁-C₁₀, un cycle C³ saturé ou non, séparé ou accolé à un autre cycle C⁴, contenant éventuellement au moins un hétéroatome, les radicaux alkyle et les cycles pouvant, en outre, être substitués par au moins un substituant A₁ où R₇ et R'₇ identiques ou différents désignent :
. l'atome d'hydrogène ou un radical alkyle en C₁-C₁₀, linéaire ou ramifié, saturé ou non,
. un cycle aromatique C₆ incluant éventuellement au moins un hétéroatome, éventuellement substitué par au moins un substituant A₂ ; et
où les hétéroatomes sont choisis parmi N, O, S et leur association. 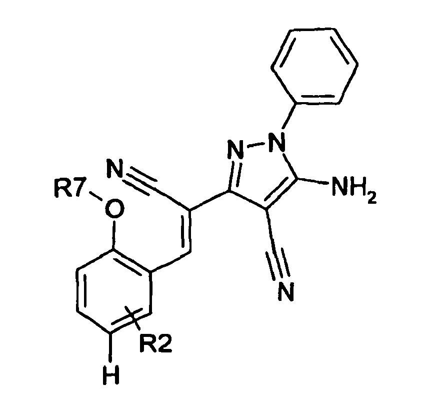 R₇ représente
a) un radical alkyle en C₁-C₁₀, linéaire ou ramifié, saturé ou non, éventuellement substitué par au moins un substituant A₁; ou
b) un cycle C¹ de 4 à 7 atomes, saturé ou insaturé, contenant éventuellement au moins un hétéroatome et/ou étant éventuellement substitué par au moins un substituant A₁ et/ou éventuellement accolé à au moins un cycle C² de 4 à 7 atomes, saturé ou insaturé, contenant éventuellement au moins un hétéroatome ;
R₂ représente
. OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, un radical alkyle, saturé ou non, en C₁-C₁₀, un cycle C³ saturé ou non, séparé ou accolé à un autre cycle C⁴, contenant éventuellement au moins un hétéroatome, les radicaux alkyle et les cycles pouvant, en outre, être substitués par au moins un substituant A₁ où R₇ et R'₇ identiques ou différents désignent :
. l'atome d'hydrogène ou un radical alkyle en C₁-C₁₀, linéaire ou ramifié, saturé ou non,
. un cycle aromatique C₅ incluant éventuellement au moins un hétéroatome, éventuellement substitué par au moins un substituant A₂; et
où les hétéroatomes sont choisis parmi N, O, S et leur association.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau y compris le cuir chevelu et les paupières et sur les fibres kératiniques d'êtres humains. Par "cosmétique", on entend au sens de l'invention une composition d'aspect, d'odeur et de toucher agréables.

Le composé de formule (I) ou l'un de ses sels peut être utilisé dans une composition qui doit être ingérée, injectée ou appliquée sur la peau ou sur les fibres kératiniques (sur toute zone cutanée ou des fibres à traiter).

Selon l'invention, le composé de formule (1) ou l'un de ses sels peut être utilisé par la voie orale en une quantité de 0,1 à 300 mg par jour, par exemple de 5 à 10mg/j.

Une composition préférée de l'invention est une composition à usage cosmétique et en particulier d'application topique sur la peau et les fibres kératiniques, et plus spécialement sur le cuir chevelu, les cheveux, les cils.

Cette composition peut se présenter sous toutes formes galéniques connues adaptées au mode d'utilisation.

Pour une application topique sur la peau, la composition peut avoir la forme d'une solution ou suspension aqueuse, alcoolique ou hydro-alcoolique ou d'une suspension huileuse, d'une émulsion de consistance plus ou moins fluide et notamment liquide ou semi-liquide, obtenue par dispersion d'une phase grasse dans une phase aqueuse (H/E) ou inversement (E/H), d'une émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique. Elle peut ainsi se présenter sous forme d'une lotion, sérum, lait, crème H/E ou E/H, d'onguent, pommade, baume, patch, tampon imbibé.

On peut également envisager une composition sous forme de mousse ou encore sous forme de spray ou d'aérosol comprenant alors un agent propulseur sous pression.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application topique sur les cils et les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire ou pour cils.

Les quantités des différents constituants de la composition selon l'invention sont celles généralement utilisées dans les domaines considérés. En outre, ces compositions sont préparées selon les méthodes usuelles. -

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100% en poids. En pratique, la phase aqueuse représente de 5 à 99,9 % du poids du poids total de la composition.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et co-émulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement pour une application capillaire, la composition est une solution ou suspension aqueuse, alcoolique ou hydro-alcoolique et mieux une solution ou suspension eau/éthanol. La fraction alcoolique peut représenter de 5 à.99,9 % et notamment de 8 à 80 %.

Pour une application mascara, la composition est une dispersion de cire-dans-eau ou de cire-dans-huile, une huile gélifiée, un gel aqueux, pigmenté ou non.

La composition de l'invention peut comprendre, en outre, d'autres ingrédients usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les polymères filmogènes, les agents bloqueurs d'U.V. comme les filtres solaires, les actifs cosmétiques et pharmaceutiques à action bénéfique pour al peau ou les fibres kératiniques, autres que les composés de formule (I) leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %.

Bien entendu l'homme du métier veillera à choisir les éventuels additifs complémentaires et/ou leur quantité de telle manière que les propriétés avantageuses de la composition selon l'invention, à savoir l'inhibition de la 15-PGDH notamment ou l'augmentation de la densité des fibres kératiniques (capillaires ou cils), ne soient pas ou substantiellement pas, altérées par l'adjonction envisagée.

Comme solvants utilisables dans l'invention, on peut citer les alcools inférieurs en C₂ à C₈ comme l'éthanol, l'isopropanol, le propylène glycol et certaines huiles cosmétiques légères comme les alcanes en C₆ à C₁₆.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, alcool ou acide gras), les huiles d'origine animale (perhydrosqualène), les huiles de synthèse (ester d'acide gras, huile de Purcellin), les huiles siliconées (polydiméthylsiloxane linéaire ou cyclique, phényltriméthicone) et les huiles fluorées (perfluoropolyéthers). Comme cires, on peut citer les cires siliconées, les cires de riz, de candellila, d'abeille, de carnauba ou paraffine, de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

La composition peut contenir, en outre, un actif cosmétique ou pharmaceutique autre que les composés de formule (1) qui peut être hydrophile et choisi parmi les protéines, les hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les-hydroxy-acides (acides de fruit ou acide salicylique) ; ou lipophile, et choisi parmi le rétinol (vitamine A) et ses dérivés notamment ester (palmitate de rétinol), le tocophérol (vitamine E) et ses dérivés (acétate de tocophérol), les acides gras essentiels, les céramides, les huiles essentielles, les dérivés de l'acide salicylique comme le n-octanoyl-5 salicylique, les esters des hydroxy-acides, les phospholipides comme la lécithine, leurs mélanges.

Selon un mode particulier de réalisation de l'invention, on peut associer au composé de formule (I) ou un de ses sels, au moins un composé actif additionnel favorisant la repousse et/ou limitant la chute des fibres kératiniques (cheveux, cils). Ces composés additionnels sont notamment choisis parmi les inhibiteurs de lipoxygénase tels que décrits dans EP 0648488, les inhibiteurs de bradykinine décrits notamment dans EP 0845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP 1175890, WO 01/74307, WO 01/74313, WO 01/74314, WO 01/74315 ou WO 01/72268, leurs mélanges.

Comme autres composés actifs additionnels favorisant la pousse des fibres kératiniques (notamment du cheveu) et/ou limitant leur chute, pouvant être présents dans la composition selon l'invention, on peut citer les vasodilatateurs, les antiandrogènes, les cyclosporines et leurs analogues, les antimicrobiens et antifongiques, les anti-inflammatoires, les rétinoïdes, leurs mélanges.

Les vasodilatateurs utilisables sont notamment les agonistes des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les anti-androgènes utilisables incluent notamment les inhibiteurs stéroïdiens et non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide, l'oxendolone, la spironolactone, le diéthylstilbestrol et les composés décrits dans les brevets US 5 411981, 5 565467 et 4 910226.

Les composés antimicrobiens ou antifongiques peuvent être choisis parmi les dérivés du sélénium, l'octopirox, le kétoconazole, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle, la minocycline et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0770399, WO 94/06434 et FR 2268523.

Les rétinoïdes peuvent être choisis parmi l'isotrétinoïne, l'acitrétine et le tazarotène.

Comme autres composés actifs pour favoriser la pousse et/ou limiter la chute des fibres kératiniques (cheveux notamment) utilisables en associations avec le composé de formule (I), on peut citer l'aminexil, le 6-O-[(9Z,12Z)-octadéca-9,12-diènoyl]hexopyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones, l'hydantoïne, l'acide rétinoïque ; les vitamines comme la vitamine D, les analogues de la vitamine B12 et le panthoténol ; les agents antiprurigineux comme la thénaldine, la triméprazine ou la cyproheptadine; les antiparasitaires, en particulier le métronidazole, le crotamiton ou les pyréthrinoïdes; les agents antagonistes de calcium, comme la cinnarizine, le diltiazem, la nimodipine, vérapamil et la nifédipine ; les hormones telles que l'estriol ou ses analogues, la thyroxine et ses sels, la progestérone ; les triterpènes comme l'acide ursolique et les composés décrits dans US 5529769, US 5468888, US 5631282 ; les agonistes du récepteur FP (récepteur aux prostaglandines du type F) tels que le latonoprost, le bimatoprost, le travoprost, l'unoprostone ; leurs mélanges.

Avantageusement, la composition selon l'invention comprendra au moins un inhibiteur de la 15-PGDH tel que défini précédemment et au moins une prostaglandine ou un dérivé de prostaglandine comme par exemple les prostaglandines de la série 2 dont notamment PGF2-α et PGE2 sous forme salines ou esters (exemple les isopropyl esters), leurs dérivés comme le 16,16 diméthyl PGE2, le 17 phényl PGE2, le 16,16 diméthyl PGF2-α, le 17 phényl PGF2-α les prostaglandines de la série 1 comme le 11 déoxy prostaglandine E1, le 1 déoxy prostaglandine E1 sous forme saline ou ester, leurs analogues notamment le latanoprost, le travoprost, le bimatoprost, le fluprosténol, le cloprosténol, le viprostol, le butaprost, le misoprostol, l'unoprostone, leurs sels ou leurs esters.

De manière préférée, la composition contient au moins un agoniste non prostanoïque des récepteurs EP2 et/ou EP4 notamment tel que décrit dans EP 1175892.

On peut également envisager que la composition comprenant au moins le composé de formule (I) ou l'un de ses sels soit sous forme liposomée, telle que notamment décrite dans le document WO 94/22468. Ainsi, le composé encapsulé dans les liposomes peut être délivré sélectivement au niveau du follicule pileux.

La composition selon l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un composé de formule (I) ou un de ses sels, le soir, garder celle-ci au contact toute la nuit et éventuellement effectuer un shampooing le matin. Ces applications peuvent être renouvelées quotidiennement pendant un ou plusieurs mois suivant les individus.

Avantageusement, dans le procédé selon l'invention, on applique sur les zones à traiter du cuir chevelu entre 5 et 500 µl d'une solution ou composition telle que définie précédemment, comprenant de 0,001% à 5 % d'inhibiteur de la 15-PGDH.

### EXEMPLES

On va maintenant donner à titre d'illustration des exemples de réalisation de l'invention qui ne sauraient limiter en aucune façon sa portée.

Comme exemples de composés pyrazoliques de formule (I) utilisables dans l'invention on peut citer les composés suivants :
Composé 1 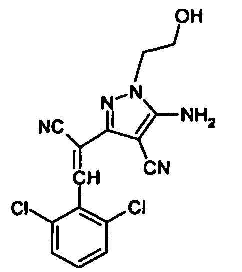 et plus spécialement le composé 1 a (cycle en position Z de la double liaison)
Composé 1a 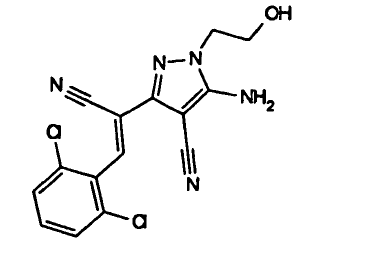
Composé 2 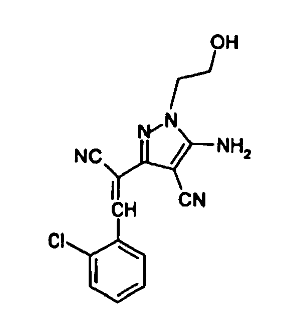
Composé 3 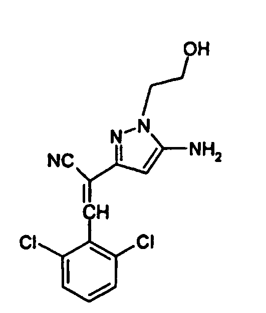
Composé 4 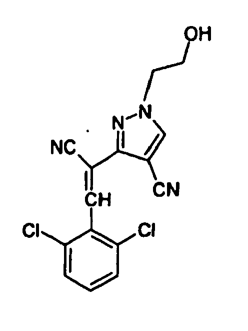
Composé 5 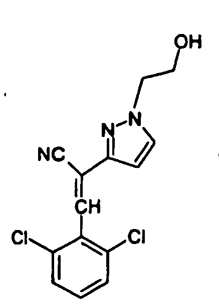
Composé 6 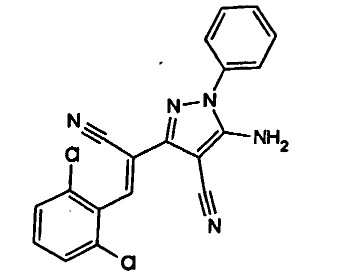
Composé 7 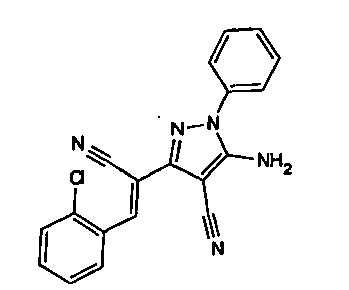
Composé 8 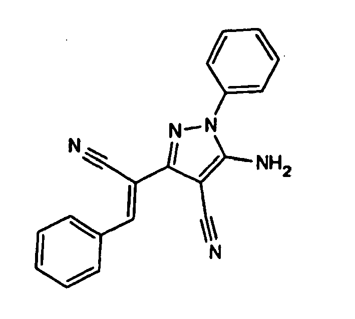
Composé 9 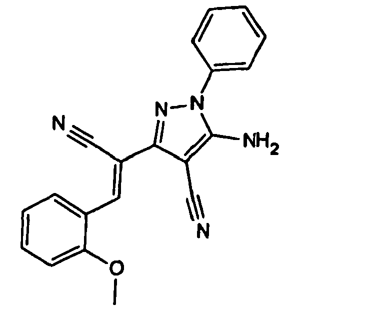
Composé 10 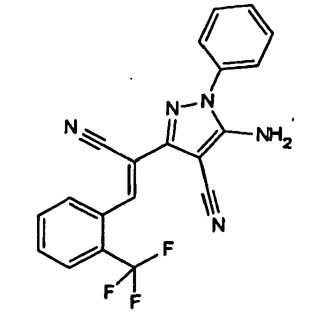 Comme composé pyrazolique nouveau de formule (1) et (III), on peut citer le composé 11
Composé 11

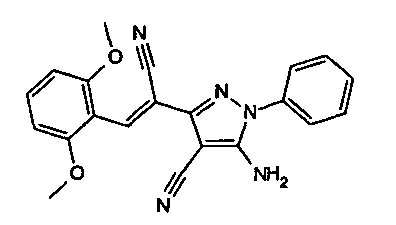

### EXEMPLE 1 : Mode opératoire de la synthèse du 5-amino-3-[1-cyano-2-(2,6-diméthoxyphényl)-vinyl]-1-phényl-1H-pyrazole-4-carbonitrile (Composé 11).

Dans un ballon sous atmosphère d'argon surmonté d'un appareil de Dean Stark, 1 g (4,48 mmol) de 5-amino-4-cyano-1-phényl-3-pyrazoleacétonitrile est mis en suspension dans 15 mL de toluène. 0,744 g (1 éq.) de 2,6-diméthoxybenzaldéhyde et 0,030 mL de pipéridine sont ajoutés au mélange. Le milieu réactionnel est chauffé à reflux pendant une nuit, puis laissé redescendre jusqu'à température ambiante. Un précipité blanchâtre se forme et est filtré et lavé au toluène. Le filtrat est concentré à sec et le résidu est repris dans l'éthanol sous agitation pendant 15 minutes. La suspension est filtrée et le filtrat concentré à sec. Le résidu est regroupé avec le précipité précédemment obtenu, et purifié sur gel de silice (éluant : dichlorométhane / méthanol 98/2). 619 mg de produit sont ainsi obtenus avec un rendement de 37 %

### Analyses :

**Sepctrométrie Masse :** (ESI +/- dans CH₃OH/H₂O) : 372(MH)⁺, 394(MNa)⁺, 743(2MH)⁺, 765(2MNa)⁺, 370(M-H)⁻:
**Résonance Magnétique Nucléaire:** :¹H (400 MHz ; DMSO-d₆) δppm : 3,85 (s, 6H, OCH₃(13) et OCH₃(9)) ; 6,78 (d, 2H, H(10) et H(12)) ; 6,95 (s, 2H, NH₂(3)) ; 7,42 à 7,57 (m, 6H, H(2') à H(6') et H(11)) ; 7,86 (s, 1H, H(7))

| **Analyse élémentaire :** | | | | |
|---|---|---|---|---|
| *Théorie :* | C 67,91 % ; | H 4,61 % ; | N 18,86% ; | O 8,62% |
| *Analyse :* | C 67,30% ; | H 4,46% ; | N 18,88% ; | O 8,96% |

### EXEMPLE 2 : Mise en évidence des propriétés inhibitrices spécifiques de la 15-PGDH des composés de formule (1).

### 1°) Test sur 15-PGDH

L'enzyme 15-PGDH est obtenue comme décrit dans la demande FR 02/05067 déposée au nom de L'Oréal, en suspension dans un milieu adapté à une concentration de 0,3 mg/ml puis bloquée à - 80°C. Pour les besoins du test, cette suspension est décongelée et-stockée dans de la glace.

Par ailleurs on prépare un tampon Tris 100 mM, pH = 7,4, contenant 0,1 mM de dithiothreitol (D5545, Sigma-Aldrich, L'isle D'Abeau Chesne, BP 701, 38297, Saint Quentin Fallavier), 1,5 mM de β-NAD (N6522, Sigma-Aldrich, L'isle D'Abeau Chesne, BP 701, 38297, Saint Quentin Fallavier), 50 µM de Prostaglandine E₂ (P4172, Sigma-Aldrich, L'isle D'Abeau Chesne, BP 701, 38297, Saint Quentin Fallavier).

Dans la cuve d'un spectrophotomètre (Perkin-Elmer, Lambda 2) thermostaté à 37°C, dont la longueur d'onde de mesure est réglée à 340 nm, sont introduits 0,965 ml de ce tampon (préalablement porté à 37°C). 0,035 ml de suspension enzymatique à 37°C sont introduits dans la cuve concomitamment à l'enregistrement (correspondant à une augmentation de la densité optique à 340 nm). La vitesse maximale de réaction est relevée.

Les valeurs essais (contenant les composés (I)) sont comparées à la valeur témoin (sans composé (I)); les résultats indiqués représentent la concentration à laquelle le composé (I) inhibe 50 % de l'activité enzymatique de 15-PGDH, notée IC_{50dh}.

### 2°) Test sur PGF Synthase

L'enzyme PGFS est obtenue comme décrit dans le document FR-A-02/05067, à la concentration de 0,5 mg/ml, en suspension dans un milieu approprié, et bloquée à -80°C. Pour les besoins du test, cette suspension est décongelée et stockée dans la glace.

Par ailleurs, on prépare dans un flacon brun (abri de la lumière) un tampon Tris 100, mM, pH = 6,5 contenant 20 µM de 9,10 phénanthrène quinone* (P2896, Sigma-Aldrich, L'isle D'Abeau Chesne, BP 701, 38297, Saint Quentin Fallavier) et 100 µM de β-NADPH (N1630, Sigma-Aldrich, L'isle D'Abeau Chesne, BP 701, 38297, Saint Quentin Fallavier).
* Une solution mère titrant 1 mM est préparée dans de l'éthanol absolu, portée à 40°C ; le flacon est placé dans une cuve à ultrason pour faciliter la solubilisation du produit.

Dans la cuve d'un spectrophotomètre (Perkin-Elmer, Lambda 2) thermostaté à 37°C, dont la longueur d'onde de mesure est réglée à 340 nm sont introduits 0,950 ml de ce tampon (préalablement porté à 37°C). 0,05 ml de suspension enzymatique à 37°C sont introduits dans la cuve concomitamment à l'enregistrement (correspondant à une baisse de la densité optique à 340 nm). La vitesse maximale de réaction est relevée.

Les valeurs essais (contenant le composé (I)) sont comparées à la valeur témoin (sans composé (I)) ; les résultats indiqués représentent la concentration à laquelle le composé (I) inhibe 50% de l'activité enzymatique de PGFS, notée IC_{50*fs*}.

| composé | structure | Inhibition de 15-PGDH IC_{50dh} µM | Inhibition de PGF synthase IC_{50fs} µM | Sélectivité |
|---|---|---|---|---|
| 1a | 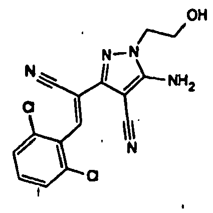 | 3 | >50 | >16,6 |
| 6 | 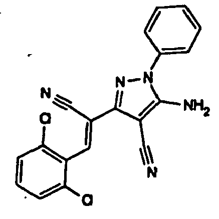 | 0,8 | >50 | >62 |
| 7 | 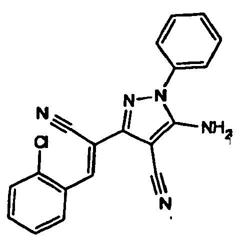 | 3 | >50 | >16 |
| 8 | 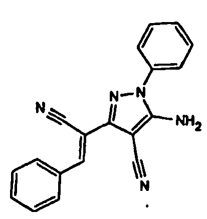 | 50 | >75 | >1,5 |
| 9 | 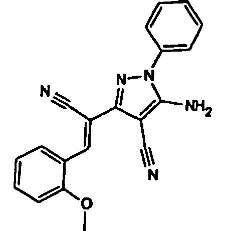 | 5 | >50 | >10 |

Il ressort de ce tableau que le rapport IC_{50fs}/IC_{50dh} des composés 1a, 6, 7, 8 et 9 est > 1,5. Les composés 1a, 6, 7, 8 et 9 et plus spécialement 1a, 6, 7 et 9 ont donc une activité inhibitrice sélective vis-à-vis de 15-PGDH par rapport à PGF synthase.

Les compositions ci-après sont obtenues par les techniques habituelles couramment utilisées dans le domaine cosmétique ou pharmaceutique.

### EXEMPLE 3 : Lotion capillaire

- Composé 1a 0,80 g
- Propylène glycol 10,00 g
- Alcool isopropylique qsp 100,00 g

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse.

### EXEMPLE 4 : Lotion capillaire

- Composé 2 1,00 g
- Propylène glycol 30,00 g
- Alcool éthylique 40,00 g
- Eau qsp 100,00 g

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre.

### EXEMPLE 5 : Lotion capillaire

- Composé 1a 1 g
- Alcool éthylique 40,00 g
- HCl qsp (*)
- Eau qsp 100,00 g
(*) quantité suffisante pour neutraliser la fonction amine portée sur le noyau pyrazole.

On applique cette lotion sur le cuir chevelu, une à deux fois par jour, à raison d'1 ml par application en massant légèrement le cuir chevelu pour faire pénétrer l'actif.

### EXEMPLE 6 : Lotion capillaire

- Composé 1a 0,10 g
- Latanoprost 0,10 g
- Propylène glycol 30,00 g
- Alcool éthylique 40,00 g
- Eau qsp 100,00 g

### EXEMPLE 7 : Mascara cire/eau

- Cire d'abeilles 6,00 %
- Cire de paraffine 13,00 %
- Huile de jojoba hydrogénée 2,00 %
- Polymère filmogène hydrosoluble 3,00 %
- Stéarate de triéthanolamine 8,00 %
- Composé 5 1,00 %
- Pigment noir 5,00 %
- Conservateur qs
- Eau qsp 100,00 %

Ce mascara s'applique sur les cils comme un mascara classique avec une brosse à mascara.

## Revendications

1. Utilisation cosmétique d'au moins un composé styryl-pyrazole de formule (1) ou d'un de ses sels, 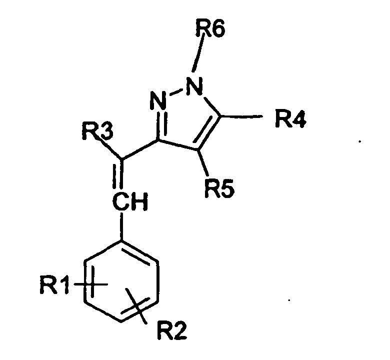 dans laquelle :
- R₁, R₂, R₄ et R₅ identiques ou différents sont choisis parmi l'hydrogène, un halogène, les groupes OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, les radicaux alkyle, saturés ou insaturés, linéaires ou ramifiés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués par au moins un substituant A₁, avec R₇, R'₇, R"₇ et R"'₇, désignant indépendamment l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₂ ;
- R₃ est choisi parmi CN, COOR₈, CONR₈R'₈, COR₈, SO₂R₈, SO₂NR₈R'₈, avec R₈ et R'₈ désignant indépendamment l'hydrogène ou un radical alkyle en C₁-C₂₀, linéaire ou ramifié ou un cycle de 4 à 7 atomes, isolé ou accolé à un autre cycle et contenant éventuellement au moins un hétéroatome, le radical alkyle ou lesdits cycles étant saturés ou non et éventuellement substitués par un au moins un substituant A₃ ;
- R₈ est choisi parmi l'hydrogène, les groupes COOR₉, COR₉, CSR₉, COSR₉, CONR₉R'₉, SO₂R₉, SO₂NR₉R'₉, les radicaux alkyle en C₁-C₂₀, linéaires ou ramifiés, saturés ou non et les cycles de 4 à 7 atomes, saturés ou insaturés, contenant éventuellement au moins un hétéroatome, ces cycles pouvant être séparés ou accolés, les radicaux alkyle et les cycles pouvant, en outre, être, substitués par au moins un substituant A₄, avec Re et R'₉ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₅ ;
- A₁, A₂, A₃, A₄ et A₅ étant indépendamment choisis parmi les halogènes, les groupes OR₁₀, SR₁₀, NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R"'₁₀, NR₁₀CSNR'₁₀R"₁₀, NR₁₀CSR'₁₀, avec R₁₀, R'₁₀, R"₁₀ et R"'₁₀ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non,
dans une composition cosmétique de soin et/ou de maquillage des fibres kératiniques humaines pour induire et/ou stimuler leur croissance, freiner leur chute et/ou augmenter leur densité.

2. Utilisation cosmétique d'au moins un composé styryl-pyrazole de formule (1) ou de l'un de ses sels, 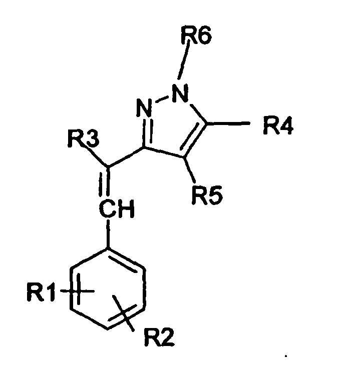 dans laquelle:
- R₁, R₂, R₄ et R₅ identiques ou différents sont choisis parmi l'hydrogène, un halogène, les groupes OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, les radicaux alkyle, saturés ou insaturés, linéaires ou ramifiés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués par au moins un substituant A₁, avec R₇, R'₇, R"₇ et R"'₇, désignant indépendamment l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₂ ;
- R₃ est choisi parmi CN, COOR₈, CONR₈R'₈ COR₈, SO₂R₈, SO₂NR₈R'₈, avec R₈ et R'₈ désignant indépendamment l'hydrogène ou un radical alkyle en C₁-C₂₀, linéaire ou ramifié ou un cycle de 4 à 7 atomes, isolé ou accolé à un autre cycle et contenant éventuellement au moins un hétéroatome, le radical alkyle ou lesdits cycles étant saturés ou non et éventuellement substitués par un au moins un substituant A₃ ;
- R₈ est choisi parmi l'hydrogène, les groupes COOR₉, COR₉, CSR₉, COSR₉, CONR₉R'₉, SO₂R₉, SO₂NR₉R'₉, les radicaux alkyle en C₁-C₂₀, linéaires ou ramifiés, saturés ou non et les cycles de 4 à 7 atomes, saturés ou insaturés, contenant éventuellement au moins un hétéroatome, ces cycles pouvant être séparés ou accolés, les radicaux alkyle et les cycles pouvant, en outre, être, substitués par au moins un substituant A₄, avec R₉ et R'₉ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₅ ;
- A₁, A₂, A₃, A₄ et A₅ étant indépendamment choisis parmi les halogènes, les groupes OR₁₀, SR₁₀, NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"10, NR₁₀C(=NR'₁₀)NR "₁₀R"'₁₀, NR₁₀CSNR'₁₀R"₁₀, NR₁₀CSR'₁₀, avec R₁₀, R'₁₀, R"₁₀ et R"'₁₀ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non,
comme inhibiteur de la 15-hydroxy prostaglandine déshydrogénase notamment humaine.

3. Utilisation selon l'une des revendications précédentes, **caractérisée en ce que** les fibres kératiniques sont les cheveux, les sourcils, les cils, les poils de barde, de moustache et les poils pubiens.

4. Utilisation d'au moins un composé styryl-pyrazole de formule (I) ou d'un de ses sels, 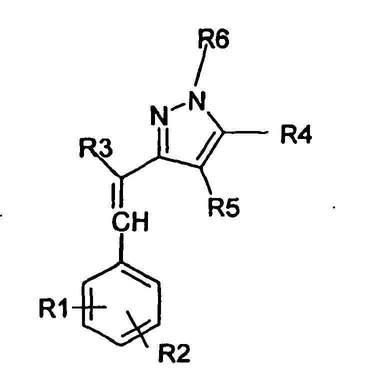 dans laquelle :
- R₁, R₂, R₄ et R₅ identiques ou différents sont choisis parmi l'hydrogène, un halogène, les groupes OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'_{7.} NR₇CONR'₇R"₇, NR₇C(=NR'₇)NR"₇R"'₇, NR₇CSR'₇, NR₇CSNR'₇R"₇, les radicaux alkyle, saturés ou insaturés, linéaires ou ramifiés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués par au moins un substituant A₁, avec R₇, R'₇, R"₇ et R"'₇, désignant indépendamment l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₂ ;
- R₃ est choisi parmi CN, COOR₈, CONR₈R'₈, COR₈, SO₂R₈, SO₂NR₈R'₈, avec R₈ et R'₈ désignant indépendamment l'hydrogène ou un radical alkyle en C₁-C₂₀, linéaire ou ramifié ou un cycle de 4 à 7 atomes, isolé ou accolé à un autre cycle et contenant éventuellement au moins un hétéroatome, le radical alkyle ou lesdits cycles étant saturés ou non et éventuellement substitués par un au moins un substituant A₃ ;
- R₆ est choisi parmi l'hydrogène, les groupes COOR₉, COR₉, CSR₉, COSR₉, CONR₉R'₉, SO₂R₉, SO₂NR₉R'₉, les radicaux alkyle en C₁-C₂₀, linéaires ou ramifiés, saturés ou non et les cycles de 4 à 7 atomes, saturés ou insaturés, contenant éventuellement au moins un hétéroatome, ces cycles pouvant être séparés ou accolés, les radicaux alkyle et les cycles pouvant, en outre, être, substitués par au moins un substituant A₄, avec R₉ et R'₉ identiques ou différents désignant l'hydrogène, un radical alkyle en C₁-C_{20'} linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₅ ;
- A₁, A₂, A₃, A₄ et A₅ étant indépendamment choisis parmi les halogènes, les groupes OR₁₀, SR₁₀, NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCR₁₀, COSR₁₀, SCOR₁₀, CSNR₁₀R'₁₀, NR₁₀CONR'₁₀R"₁₀. NR₁₀C(=NR'₁₀)NR"₁₀R'"₁₀, NR₁₀CSNR'₁₀R"₁₀, NR₁₀CSR"₁₀, avec R₁₀, R'₁₀, R"₁₀ et R"'₁₀ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non,
pour la préparation d'une composition capillaire d'être humain, destinée à traiter l'alopécie androgénique et/ou traiter l'alopécie naturelle.

5. Utilisation selon l'une des revendications précédentes, **caractérisée en ce que** le composé styryl-pyrazole présente la formule (II) suivante ou l'un de ses sels.: 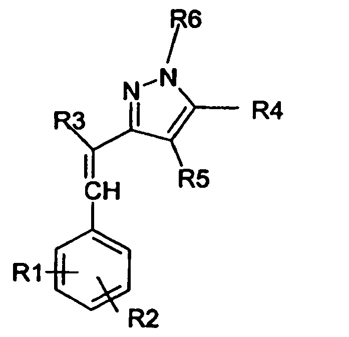 dans laquelle :
- R₁, R₂, R₄ et R₅ représentent indépendamment H, un halogène, OR₇, SR₇, NR₇R'₇, COOR₇, CONR₇R'₇, CF₃, CN, un radical alkyle, saturé ou non, en C₁-C₁₀, un cycle saturé ou non, séparé ou accolé à un autre cycle, contenant éventuellement au moins un hétéroatome, les radicaux alkyle et les cycles pouvant, en outre, être substitués par au moins un substituant A₁, avec R₇, R₇' désignant indépendamment H, un radical alkyle en C₁-C₁₀ ou un cycle isolé ou accolé à un autre cycle;
- R₃ représente CN, COOR₈, CONR₈R'₈, COR₈, avec R₈ et R'₈ désignant indépendamment H, un radical alkyle en C₁-C₁₀ ou un cycle isolé ou accolé à un autre cycle et contenant éventuellement au moins un hétéroatome, lesdits cycles étant saturés ou non et éventuellement substitués par au moins un substituant A₁ ;
- R₆ représente l'hydrogène, COOR₉, COR₉, un radical alkyle en C₁-C₁₀ saturé ou non ou un cycle saturé ou non, séparé ou accolé à un autre cycle, contenant éventuellement au moins un hétéroatome, les radicaux alkyle et les cycles pouvant, en outre, être, substitués par au moins un substituant A₁, avec R₉ et R'₉ désignant indépendamment H, un radical alkyle en C₁-C₂₀ ou un cycle isolé ou accolé à un autre cycle ;
- les cycles ayant de 5 à 6 atomes,
- les hétéroatomes étant O N, S ou leur association.

6. Utilisation selon l'une des revendications précédentes, **caractérisée en ce que** l'un au moins des R₁ et R₂ représentent un atome d'hydrogène, un atome d'halogène, OR₇ ou CF₃.

7. Utilisation selon l'une des revendications précédentes, **caractérisée en ce que** R₁ et R₂ sont situés sur le cycle phényle, en position ortho du branchement de la partie pyrazole.

8. Utilisation selon l'une des revendications précédentes, **caractérisée en ce que** R₁ et/ou R₂ représentent un atome d'halogène et notamment un atome de chlore.

9. Utilisation selon l'une des revendications précédentes, **caractérisée en ce que** R₃ représente CN.

10. Utilisation selon la revendication précédente, **caractérisée en ce que** R₄, R₅, R₆ représentent indépendamment l'un de l'autre NH₂, H, CN, un radical alkyle en C₁-C₁₀ éventuellement substitué par OR₁₀, un cycle hydrocarboné à 5 ou 6 atomes, saturé ou non.

11. Utilisation selon l'une des revendications précédentes, **caractérisée en ce que** R₆ représente CH₂CH₂OH ou un radical phényle.

12. Utilisation selon l'une des revendications précédentes, **caractérisée en ce que** R₄ représente NH₂ ou H.

13. Utilisation selon l'une des revendications précédentes, **caractérisée en ce que** R₅ représente CN ou H.

14. Utilisation selon l'une des revendications précédentes, **caractérisée en ce que** le composé styryl-pyrazole présente la formule (III) suivante ou l'un de ses sels. 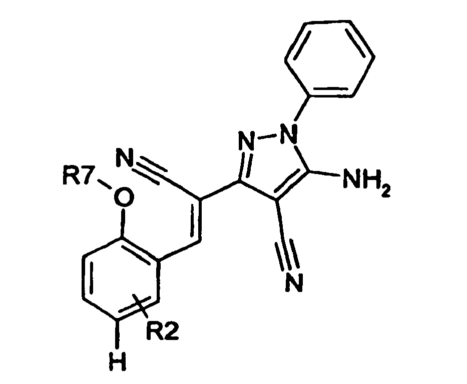 R₇ représente
a) un radical alkyle en C₁-C₁₀, linéaire ou ramifié, saturé ou non, éventuellement substitué par au moins un substituant A₁; ou
b) un cycle C¹ de 4 à 7 atomes, saturé ou insaturé, contenant éventuellement au moins un hétéroatome et/ou étant éventuellement substitué par au moins un substituant A₁ et/ou éventuellement accolé à au moins un cycle C² de 4 à 7 atomes, saturé ou insaturé, contenant éventuellement au moins un hétéroatome ;
R₂ représente
. OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇. NR₇CSNR'₇R"₇, un radical alkyle, saturé ou non, en C₁-C₁₀, un cycle C³ saturé ou non, séparé ou accolé à un autre cycle C⁴, contenant éventuellement au moins un hétéroatome, les radicaux alkyle et les cycles pouvant, en outre, être substitués par au moins un substituant A₁ où R₇ et R'₇ identiques ou différents désignent :
. l'atome d'hydrogène ou un radical alkyle en C₁-C₁₀, linéaire ou ramifié, saturé ou non,
. un cycle aromatique C⁵ incluant éventuellement au moins un hétéroatome, éventuellement substitué par au moins un substituant A₂ ;
où les hétéroatomes sont choisis parmi N, O, S et leur association.

15. Utilisation selon l'une des revendications précédentes, **caractérisée en ce que** le sel du composé de formule (I) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), d'ammonium, de manganèse (Mn²⁺), les sels de tri-éthanolamine, mono-éthanolamine, di-éthanolamine, hexadécylamine, N,N,N',N'-tétrakis-(hydroxy-propyl-2) éthylène di-amine, tris-hydroxyméthyl aminométhane, les hydroxydes, les carbonates, les sulfates, les phosphates, les halogénures, les nitrates.

16. Utilisation selon l'une des revendications précédentes, **caractérisée en ce que** le composé de formule (I) est choisi parmi :
Composé 1 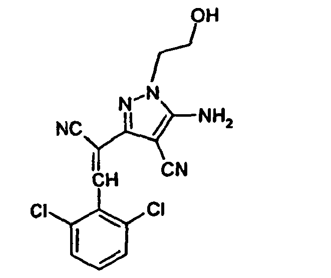
Composé 2 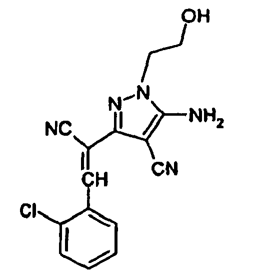
Composé 3 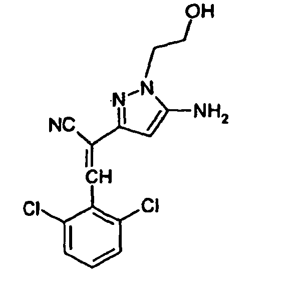
Composé 4 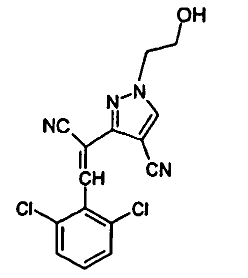
Composé 5 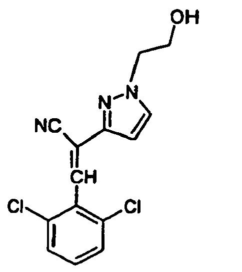
Composé 6 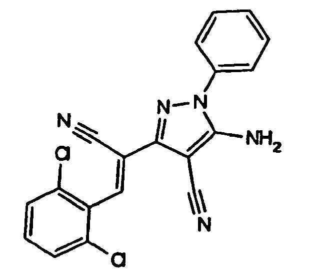
Composé 7 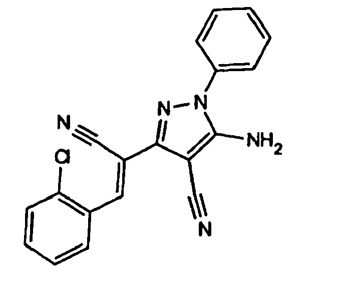
Composé 8 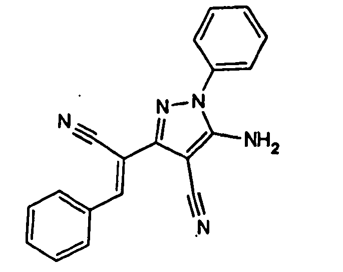
Composé 9 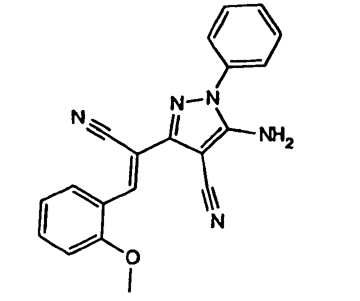
Composé 10 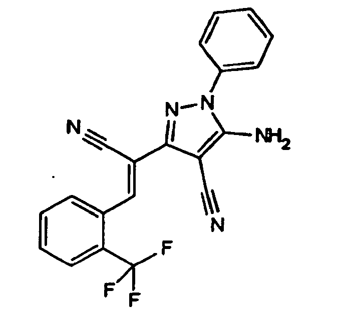
Composé 11

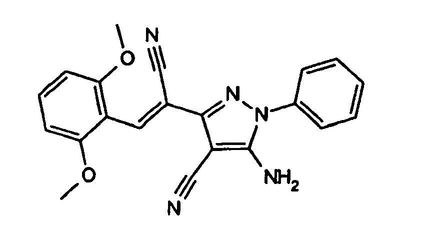

17. Utilisation selon l'une des revendications précédentes, **caractérisée en ce que** le composé de formule (I) ou un mélange de composés de formule (I) est utilisé à une concentration allant de 10⁻³ à 10%, de préférence de 10⁻² à 2%, par rapport au poids total de la composition.

18. Utilisation selon l'une des revendications précédentes, **caractérisée en ce que** la composition est une composition à application topique.

19. Composition de soin capillaire ou de maquillage des fibres kératiniques, contenant un milieu physiologiquement acceptable et une quantité efficace d'au moins un composé styryl-pyrazole de formule (1) ou de l'un de ses sels **telle que définie dans l'une quelconque des revendications 1 à 16 où R1 et R2 sont situés sur le cycle phényle, en position ortho du branchement de la partie pyrazole.**

20. Composition selon la revendication 19, **caractérisée en ce que** R₁ et/ou R₂ représentent un atome d'halogène et notamment un atome de chlore.

21. Composition selon l'une des revendications 19 et 20, **caractérisée en ce que** R₃ représente CN.

22. Composition selon l'une des revendications 19 à 21, **caractérisée en ce que** R₄, R₅, R₆ représentent indépendamment l'un de l'autre NH₂, H, CN, un radical alkyle en C₁-C₁₀ éventuellement substitué par OR₁₀, un cycle hydrocarboné à 5 ou 6 atomes, saturé ou non.

23. Composition selon l'une des revendications 19 à 22, **caractérisée en ce que** R₆ représente CH₂CH₂OH ou un radical phényle.

24. Composition selon l'une des revendications 19 à 23, **caractérisée en ce que** R₄ représente NH₂ ou H.

25. Composition selon l'une des revendications 19 à 24, **caractérisée en ce que** R₅ représente CN ou H.

26. Composition selon l'une des revendications 19 à 25, contenant un milieu physiologiquement acceptable et une quantité efficace d'au moins un composé styryl-pyrazole de formule (III) ou de l'un de ses sels : 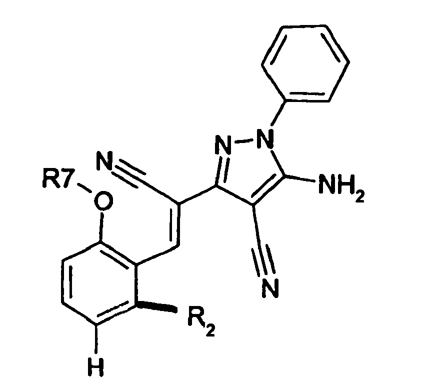 R₇ représente
a) un radical alkyle en C₁-C₁₀, linéaire ou ramifié, saturé ou non, éventuellement substitué par au moins un substituant A₁; ou
b) un cycle C¹ de 4 à 7 atomes, saturé ou insaturé, contenant éventuellement au moins un hétéroatome et/ou étant éventuellement substitué par au moins un substituant A₁ et/ou éventuellement accolé à au moins un cycle C² de 4 à 7 atomes, saturé ou insaturé, contenant éventuellement au moins un hétéroatome ;
R₂ représente
. OR₇,SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, un radical alkyle, saturé ou non, en C₁-C₁₀, un cycle C³ saturé ou non, séparé ou accolé à un autre cycle C⁴, contenant éventuellement au moins un hétéroatome, les radicaux alkyle et les cycles pouvant, en outre, être substitués par au moins un substituant A₁ où R₇ et R'₇ identiques ou différents désignent :
. l'atome d'hydrogène ou un radical alkyle en C₁-C₁₀, linéaire ou ramifié, saturé ou non,
. un cycle aromatique C₅ incluant éventuellement au moins un hétéroatome, éventuellement substitué par au moins un substituant A₂ ; et
où les hétéroatomes sont choisis parmi N, O, S et leur association.

27. Composition selon l'une des revendications 19 à 26, **caractérisée en ce que** le sel du composé de formule (I) ou (III) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), d'ammonium, les sels de tri-éthanolamine, mono-éthanolamine, di-éthanolamine, hexadécylamine, N,N,N',N'-tétrakis-(hydroxy-propyl-2) éthylène di-amine, tris-hydroxyméthyl aminométhane, les hydroxydes, les carbonates, les sulfates, les phosphates, les halogénures, les nitrates.

28. Composition selon l'une des revendications 19 à 27, **caractérisée en ce que** le composé de formule (1) est choisi parmi :
Composé 1 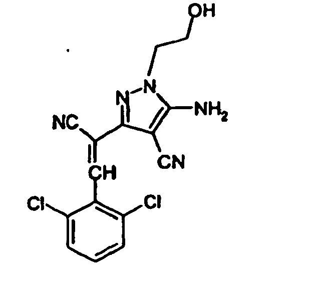
Composé 2 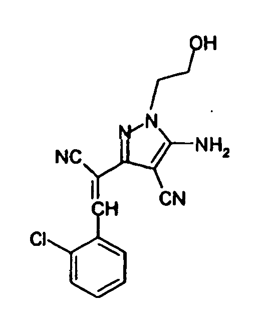
Composé 3 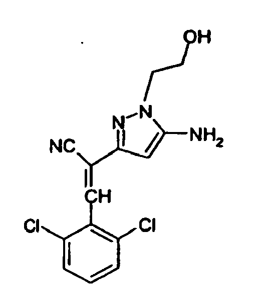
Composé 4 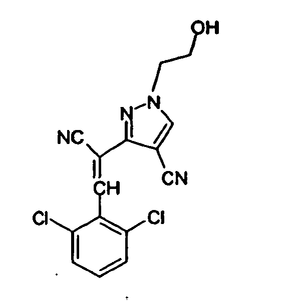
Composé 5 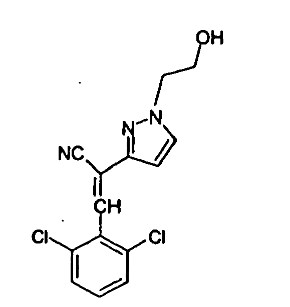
Composé 6 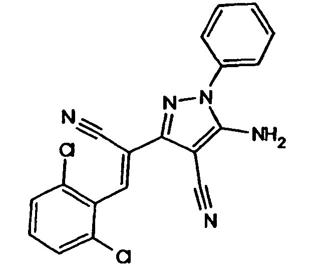
Composé 7 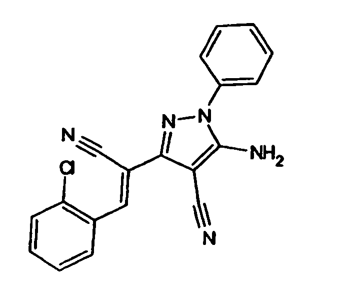
Composé 8 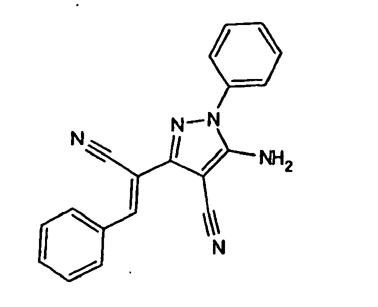
Composé 9 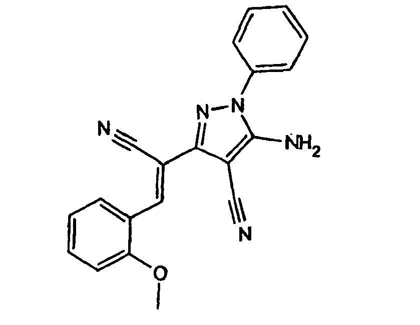
Composé 10 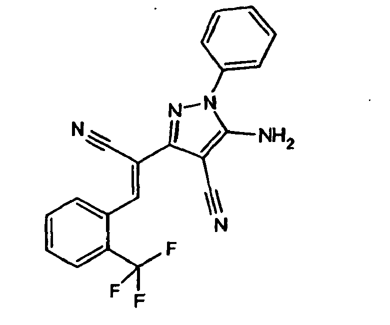
Composé 11

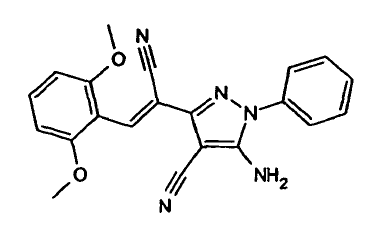

29. Composition selon l'une des revendications 19 à 28, **caractérisée en ce que** le composé de formule (1) ou un mélange de composés de formule (I) est utilisé à une concentration allant de 10⁻³ à 10%, de préférence de 10⁻² à 2%, par rapport au poids total de la composition.

30. Composition selon l'une des revendications 19 à 29, **caractérisée en ce qu'**elle se présente sous forme de crème ou lotion capillaire, de shampooing ou d'après-shampooing, de mascara capillaire ou de cils.

31. Composition selon l'une des revendications 19 à 30, **caractérisée en ce qu'**elle est sous forme de solution ou suspension aqueuse, alcoolique ou hydro-alcoolique.

32. Composition selon l'une des revendications 19 à 31, **caractérisée en ce qu'**elle contient d'autres ingrédients choisis parmi les solvants, les épaississants ou gélifiants de phase aqueuse ou de phase huileuse, les matières colorantes solubles dans le milieu de la composition, les charges, les pigments, les antioxydants, les conservateurs, les parfums, les électrolytes, les neutralisants, les polymères filmogènes, les agents bloqueurs d'U.V., les actifs cosmétiques et pharmaceutiques, leurs mélanges.

33. Composition selon l'une des revendications 19 à 32, **caractérisée en ce qu'**elle contient, en outre, un autre actif choisi parmi les protéines, les hydrolysats de protéine, les acides aminés, les polyols, l'urée, l'allantoïne, les sucres et dérivés de sucre, les extraits végétaux, les hydroxy-acides, les dérivés du rétinol ou du tocophérol, les acides gras essentiels, les céramides, les huiles essentielles, l'acide salicylique et ses dérivés comme le n-octanoyl-5 salicylique, les esters des hydroxy-acides, les phospholipides.

34. Composition selon l'une des revendications 19 à 33, **caractérisée en ce qu'**elle contient au moins un composé additionnel actif favorisant la repousse et/ou limitant la chute des fibres kératiniques.

35. Composition selon l'une des revendications 19 à 34, **caractérisée en ce qu'**elle contient au moins un composé actif additionnel favorisant la repousse et/ou limitant la chute des fibres kératiniques choisi parmi l'aminexil, le 6-*O*-[(9Z,12Z)-octadéca-9,12-diènoyl] hexapyranose, les inhibiteurs de lipoxygénase, les inhibiteurs de bradykinine, les prostaglandines et leurs dérivés, les agonistes ou antagonistes des récepteurs des prostaglandines, les analogues non prostanoïques de prostaglandines, les vasodilatateurs, les antiandrogènes, les cyclosporines et leurs analogues, les antimicrobiens, les anti-inflammatoires, les rétinoïdes, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones, l'hydantoïne, l'octopirox, l'acide rétinoïque, les agents antiprurigineux, les antiparasitaires, les antifongiques, les esters d'acide nicotinique, les agents antagonistes de calcium, les hormones, les triterpènes, les agents antiandrogènes, les inhibiteurs stéroïdiens ou non stéroïdiens des 5-α-réductases, les agonistes de canaux potassium, les agonistes du récepteur FP, leurs mélanges.

36. Composition selon la revendication 35, **caractérisée en ce que** le composé additionnel est choisi parmi l'aminexil, les agonistes du récepteur FP et les vasodilatateurs.

37. Composition selon l'une des revendications 34 à 36, **caractérisée en ce que** le composé actif additionnel est choisi parmi l'aminexil, le minoxidil, le latanoprost, le butaprost et le travoprost.

38. Procédé de traitement cosmétique des fibres kératiniques et/ou de la peau d'où émergent lesdites fibres, **caractérisé par le fait qu'**il consiste à appliquer sur les fibres et/ou la peau, une composition cosmétique telle que définie dans l'une quelconque des revendications 19 à 37, à laisser celle-ci en contact avec les fibres et/ou la peau, et éventuellement à rincer.

39. Procédé de soin cosmétique et/ou de maquillage des cils humains, en vue d'améliorer leur état et/ou leur aspect, **caractérisé en ce qu'**il consiste à appliquer sur les cils et/ou les paupières une composition telle que définie dans l'une quelconque des revendications 19 à 37 sous forme de mascara et à laisser celle-ci au contact des cils et/ou les paupières.

40. Procédé de soin cosmétique des cheveux et/ou du cuir chevelu humains, en vue d'améliorer leur état et/ou leur aspect, **caractérisé en ce qu'**il consiste à appliquer sur les cheveux et/ou le cuir chevelu, une composition cosmétique telle que définie dans l'une quelconque des revendications 19 à 37, à laisser celle-ci en contact avec les cheveux et/ou le cuir chevelu, et éventuellement à rincer.

## Claims

1. Cosmetic use of at least one styrylpyrazole compound of formula (I), or a salt thereof: 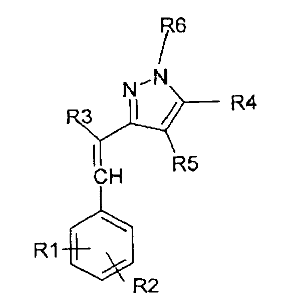 in which:
- R₁, R₂, R₄ and R₅, which may be identical or different, are chosen from hydrogen, a halogen, groups OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇ and NR₇CSNR'₇R"₇, saturated or unsaturated, linear or branched C₁-C₂₀ alkyl radicals, saturated or unsaturated rings of 4 to 7 atoms, optionally containing at least one hetero atom, these rings possibly being separate or fused, the alkyl radicals and the rings also possibly being substituted with at least one substituent A₁, with R₇, R'₇, R"₇ and R"'₇ independently denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated and optionally substituted with at least one substituent A₂;
- R₃ is chosen from CN, COOR₈, CONR₈R'₈, COR₈, SO₂R₈ and SO₂NR₈R'₈, with R₈ and R'₈ independently denoting hydrogen, a linear or branched C₁-C₂₀ alkyl radical or a ring of 4 to 7 atoms, isolated or fused to another ring and optionally containing at least one hetero atom, the alkyl radical or the said rings being saturated or unsaturated and optionally substituted with at least one substituent A₃;
- R₆ is chosen from hydrogen, groups COOR₉, COR₉, CSR₉, COSR₉, CONR₉R'₉, SO₂R₉ and SO₂NR₉R'₉, linear or branched, saturated or unsaturated C₁-C₂₀ alkyl radicals and saturated or unsaturated rings of 4 to 7 atoms, optionally containing at least one hetero atom, these rings possibly being separate or fused, the alkyl radicals and the rings also possibly being substituted with at least one substituent A₄, with R₉ and R'₉, which may be identical or different, denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated and optionally substituted with at least one substituent A₅;
- A_{1'} A₂, A₃, A₄ and A₅ being chosen independently from halogens, groups OR₁₀, SR₁₀, NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R'"₁₀, NR₁₀CSNR' ₁₀R" ₁₀ and NR₁₀CSR'₁₀, with R₁₀, R'₁₀, R"₁₀ and R"₁₀, which may be identical or different, denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated,
in a cosmetic care and/or makeup composition for human keratin fibres, to induce and/or stimulate their growth, reduce their loss and/or increase their density.

2. Cosmetic use of at least one styrylpyrazole compound of formula (I), or a salt thereof: 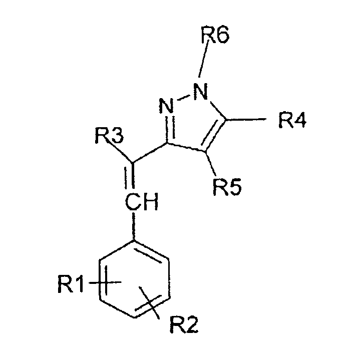 in which:
- R₁, R₂, R₄ and R₅, which may be identical or different, are chosen from hydrogen, a halogen, groups OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇ and NR₇CSNR'₇R"₇, saturated or unsaturated, linear or branched C₁-C₂₀ alkyl radicals, saturated or unsaturated rings of 4 to 7 atoms, optionally containing at least one hetero atom, these rings possibly being separate or fused, the alkyl radicals and the rings also possibly being substituted with at least one substituent A₁, with R₇, R'₇, R"₇ and R"'₇ independently denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated and optionally substituted with at least one substituent A₂;
- R₃ is chosen from CN, COOR₈, CONR₈R'₈, COR₈, SO₂R₈ and SO₂NR₈R'₈, with R₈ and R' ₈ independently denoting hydrogen, a linear or branched C₁-C₂₀ alkyl radical or a ring of 4 to 7 atoms, isolated or fused to another ring and optionally containing at least one hetero atom, the alkyl radical or the said rings being saturated or unsaturated and optionally substituted with at least one substituent A₃;
- R₆ is chosen from hydrogen, groups COOR₉, COR₉, CSR₉, COSR₉, CONR₉R'₉, SO₂R₉ and SO₂NR₉R'₉, linear or branched, saturated or unsaturated C₁-C₂₀ alkyl radicals and saturated or unsaturated rings of 4 to 7 atoms, optionally containing at least one hetero atom, these rings possibly being separate or fused, the alkyl radicals and the rings also possibly being substituted with at least one substituent A₄, with R₉ and R'₉, which may be identical or different, denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated and optionally substituted with at least one substituent A₅;
- A₁, A₂, A₃, A₄ and A₅ being chosen independently from halogens, groups OR₁₀, SR₁₀, NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C (=NR'₁₀) NR"₁₀R'"₁₀, NR₁₀CSNR'₁₀R"₁₀ and NR₁₀CSR'₁₀, with R₁₀, R'₁₀, R"₁₀ and R"'₁₀, which may be identical or different, denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated,
as an inhibitor of 15-hydroxyprostaglandin dehydrogenase, especially human 15-hydroxyprostaglandin dehydrogenase.

3. Use according to either of the preceding claims, **characterized in that** the keratin fibres are head hair, the eyebrows, the eyelashes, beard hair, moustache hair and pubic hair.

4. Use of at least one styrylpyrazole compound of formula (I), or a salt thereof: 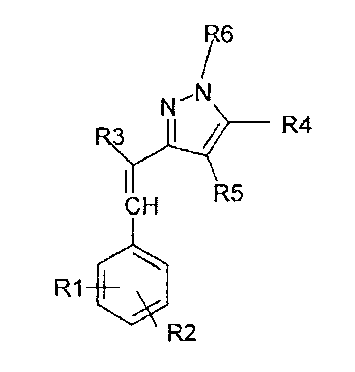 in which:
- R₁, R₂, R₄ and R₅, which may be identical or different, are chosen from hydrogen, a halogen, groups OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇ and NR₇CSNR'₇R"₇, saturated or unsaturated, linear or branched C₁-C₂₀ alkyl radicals, saturated or unsaturated rings of 4 to 7 atoms, optionally containing at least one hetero atom, these rings possibly being separate or fused, the alkyl radicals and the rings also possibly being substituted with at least one substituent A₁, with R₇, R'₇, R"₇ and R"'₇ independently denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated and optionally substituted with at least one substituent A₂;
- R₃ is chosen from CN, COOR₈, CONR₈R'₈, COR₈, SO₂R₈ and SO₂NR₈R'₈, with R₈ and R'₈ independently denoting hydrogen, a linear or branched C₁-C₂₀ alkyl radical or a ring of 4 to 7 atoms, isolated or fused to another ring and optionally containing at least one hetero atom, the alkyl radical or the said rings being saturated or unsaturated and optionally substituted with at least one substituent A₃;
- R₆ is chosen from hydrogen, groups COOR₉, COR₉, CSR₉, COSR₉, CONR₉R'₉, SO₂R₉ and SO₂NR₉R'₉, linear or branched, saturated or unsaturated C₁-C₂₀ alkyl radicals and saturated or unsaturated rings of 4 to 7 atoms, optionally containing at least one hetero atom, these rings possibly being separate or fused, the alkyl radicals and the rings also possibly being substituted with at least one substituent A₄, with R₉ and R'₉, which may be identical or different, denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated and optionally substituted with at least one substituent A₅;
- A₁, A₂, A₃, A₄ and A₅ being chosen independently from halogens, groups OR₁₀, SR₁₀, NR₁₀R'₁₀, COOR₁₀, CH₂COOR₁₀, CONR₁₀R'_{10,} CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R'"₁₀, NR₁₀CSNR'₁₀R"₁₀ and NR₁₀CSR'₁₀, with Rio, R'₁₀, R"₁₀ and R"'₁₀, which may be identical or different, denoting hydrogen, a linear or branched C₁-C₂₀ alkyl radical or a ring of 4 to 7 atoms, optionally containing at least one hetero atom, isolated or fused to another ring, the alkyl radical or the said rings being saturated or unsaturated,
for the preparation of a human hair composition, which is intended to treat androgenic alopecia and/or treat natural alopecia.

5. Use according to one of the preceding claims, **characterized in that** the styrylpyrazole compound is of formula (II) below, or a salt thereof: 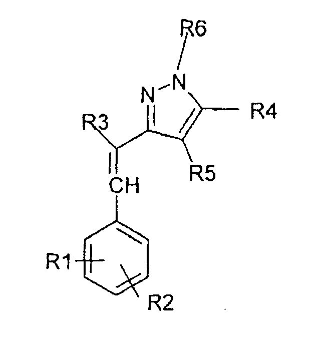 in which:
- R₁, R₂, R₄ and R₅ independently represent H, a halogen, OR₇, SR₇, NR₇R'₇, COOR₇, CONR₇R'₇, CF₃, CN, a saturated or unsaturated C₁-C₁₀ alkyl radical, a saturated or unsaturated ring, separate or fused to another ring, optionally containing at least one hetero atom, the alkyl radicals and the rings also possibly being substituted with at least one substituent A₁, with R₇ and R'₇ independently denoting H, a C₁-C₁₀ alkyl radical or a ring which is isolated or fused to another ring;
- R₃ represents CN, COOR₈, CONR₈R'₈ or COR₈, with R₈ and R'₈ independently denoting H, a C₁-C₁₀ alkyl radical or a ring which is isolated or fused to another ring and optionally containing at least one hetero atom, the said rings being saturated or unsaturated and optionally substituted with at least one substituent A₁;
- R₆ represents hydrogen, COOR₉, COR₉, a saturated or unsaturated C₁-C₁₀ alkyl radical or a saturated or unsaturated ring, which is separate or fused to another ring, optionally containing at least one hetero atom, the alkyl radicals and the rings also possibly being substituted with at least one substituent A₁, with R₉ and R'₉ independently denoting H, a C₁-C₂₀ alkyl radical or a ring which is isolated or fused to another ring;
- the rings containing 5 or 6 atoms;
- the hetero atoms being 0, N or S or a combination thereof.

6. Use according to one of the preceding claims, **characterized in that** at least one from among R₁ and R₂ represents a hydrogen atom, a halogen atom, OR₇ or CF₃.

7. Use according to one of the preceding claims, **characterized in that** R₁ and R₂ are located on the phenyl ring, in an ortho position to the branching of the pyrazole portion.

8. Use according to one of the preceding claims, **characterized in that** R₁ and/or R₂ represent(s) a halogen atom, especially a chlorine atom.

9. Use according to one of the preceding claims, **characterized in that** R₃ represents CN.

10. Use according to the preceding claim, **characterized in that** R₄, R₅ and R₆ represent, independently of each other, NH₂, H, CN, a C₁-C₁₀ alkyl radical optionally substituted with OR₁₀, or a saturated or unsaturated hydrocarbon-based ring containing 5 or 6 atoms.

11. Use according to one of the preceding claims, **characterized in that** R₆ represents CH₂CH₂OH or a phenyl radical.

12. Use according to one of the preceding claims, **characterized in that** R₄ represents NH₂ or H.

13. Use according to one of the preceding claims, **characterized in that** R₅ represents CN or H.

14. Use according to one of the preceding claims, **characterized in that** the styrylpyrazole compound is of formula (III) below, or a salt thereof: 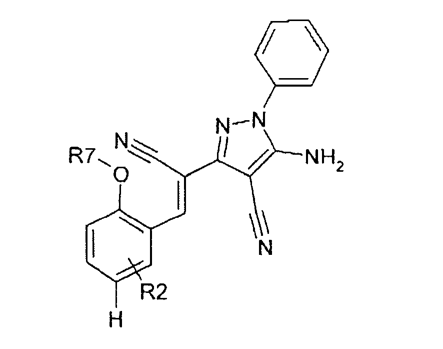 R₇ represents
a) a linear or branched, saturated or unsaturated C₁-C₁₀ alkyl radical, optionally substituted with at least one substituent A₁; or
b) a saturated or unsaturated ring C¹ of 4 to 7 atoms, optionally containing at least one hetero atom and/or being optionally substituted with at least one substituent A₁ and/or optionally fused to at least one saturated or unsaturated ring C² of 4 to 7 atoms, optionally containing at least one hetero atom;
R₂ represents
• OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C (=NR'₇)NR"₇R"'₇, NR₇CSR'₇, NR₇CSNR'₇R"₇, a saturated or unsaturated C₁-C₁₀ alkyl radical, a saturated or unsaturated ring C³, which is separate or fused to another ring C⁴, optionally containing at least one hetero atom, the alkyl radicals and the rings also possibly being substituted with at least one substituent A₁ in which R₇ and R'₇, which may be identical or different, denote:
• a hydrogen atom or a linear or branched, saturated or unsaturated C₁-C₁₀ alkyl radical,
• an aromatic ring C₅ optionally including at least one hetero atom, optionally substituted with at least one substituent A₂;
in which the hetero atoms are chosen from N, 0 and S and a combination thereof.

15. Use according to one of the preceding claims, **characterized in that** the salt of the compound of formula (I) is a salt chosen from the sodium and potassium salts, the zinc (Zn²⁺), calcium (Ca²⁺) copper (Cu²⁺), iron (Fe²⁺), strontium (Sr²⁺), magnesium (Mg²⁺), ammonium and manganese (Mn²⁺) salts, the triethanolamine, monoethanolamine, diethanolamine, hexadecylamine, N,N,N',N'-tetrakis(2-hydroxypropyl)ethylenediamine and tris(hydroxymethyl)aminomethane salts, and the hydroxides, carbonates, sulphates, phosphates, halides and nitrates.

16. Use according to one of the preceding claims, **characterized in that** the compound of formula (I) is chosen from:
Compound 1 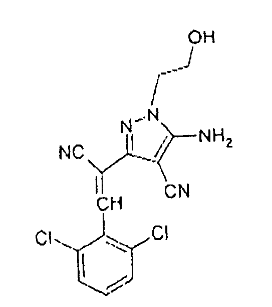
Compound 2 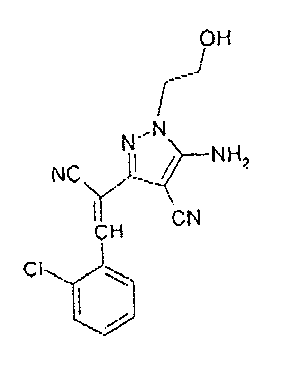
Compound 3 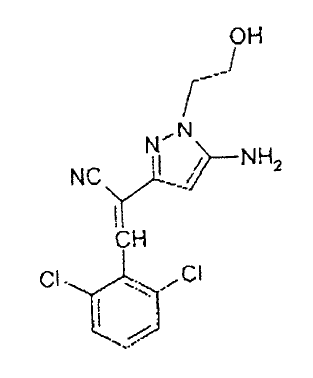
Compound 4 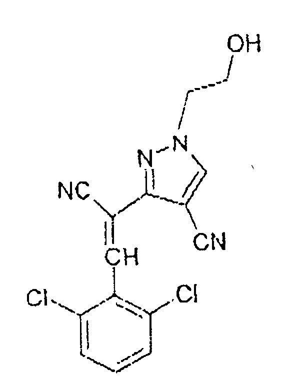
Compound 5 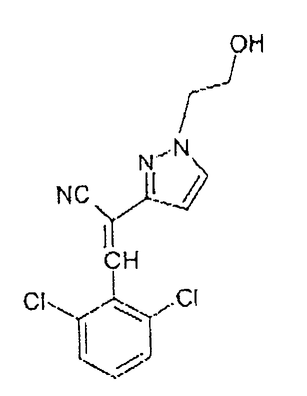
Compound 6 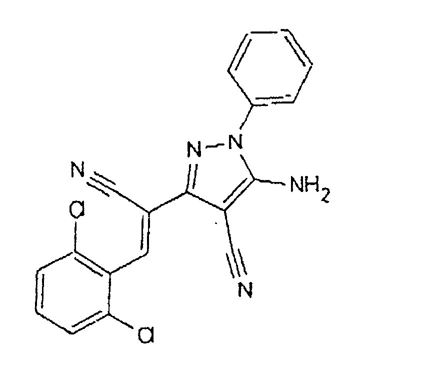
Compound 7 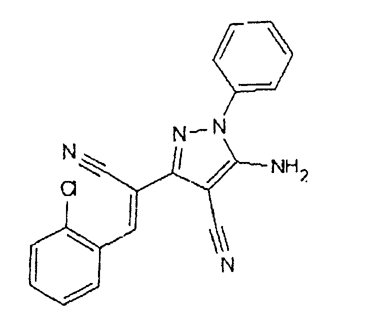
Compound 8 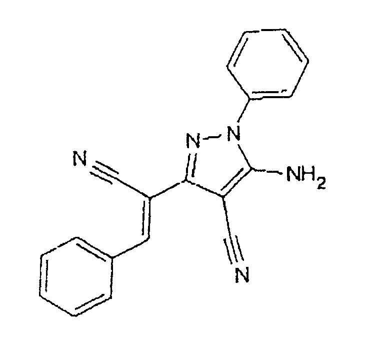
Compound 9 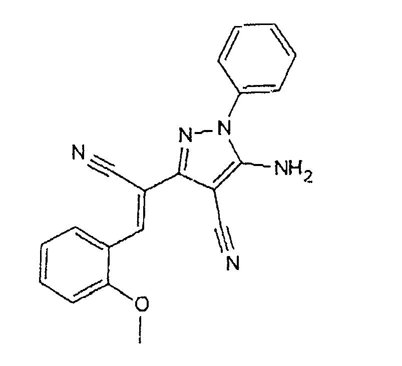
Compound 10 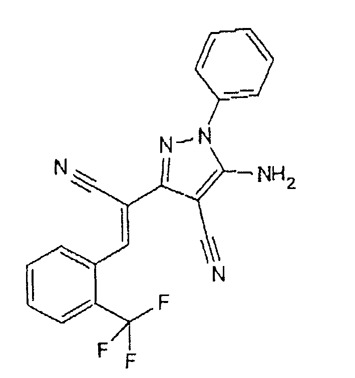
Compound 11

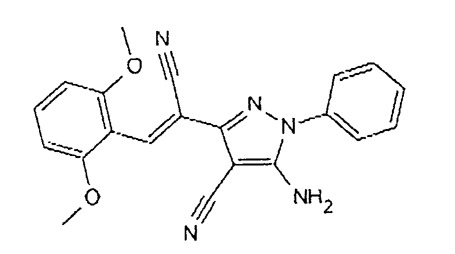

17. Use according to one of the preceding claims, **characterized in that** the compound of formula (I) or a mixture of compounds of formula (I) is used at a concentration ranging from 10⁻³% to 10% and preferably from 10⁻²% to 2% relative to the total weight of the composition.

18. Use according to one of the preceding claims, **characterized in that** the composition is a composition for topical application.

19. Haircare or makeup composition for keratin fibres, containing a physiologically acceptable medium and an effective amount of at least one styrylpyrazole compound of formula (I), or a salt thereof, as defined in any one of claims 1 to 16, where R₁ and R₂ are located on the phenyl ring, in an ortho position to the branching of the pyrazole portion.

20. Composition according to Claim 19, **characterized in that** R₁ and/or R₂ represent(s) a halogen atom, especially a chlorine atom.

21. Composition according to either of Claims 19 and 20, **characterized in that** R₃ represents CN.

22. Composition according to one of Claims 19 to 21, **characterized in that** R₄, R₅ and R₆ represent, independently of each other, NH₂, H, CN, a C₁-C₁₀ alkyl radical optionally substituted with OR₁₀, or a saturated or unsaturated hydrocarbon-based ring containing 5 or 6 atoms.

23. Composition according to one of Claims 19 to 22, **characterized in that** R₆ represents CH₂CH₂OH or a phenyl radical.

24. Composition according to one of Claims 19 to 23, **characterized in that** R₄ represents NH₂ or H.

25. Composition according to one of Claims 19 to 24, **characterized in that** R₅ represents CN or H.

26. Composition according to one of Claims 19 to 25, containing a physiologically acceptable medium and an effective amount of at least one styrylpyrazole compound of formula (III) or of a salt thereof: 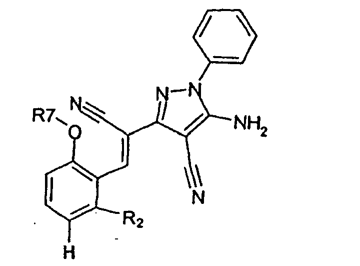 R₇ represents
a) a linear or branched, saturated or unsaturated C₁-C₁₀ alkyl radical, optionally substituted with at least one substituent A₁; or
b) a saturated or unsaturated ring C¹ of 4 to 7 atoms, optionally containing at least one hetero atom and/or being optionally substituted with at least one substituent A₁ and/or optionally fused to at least one saturated or unsaturated ring C² of 4 to 7 atoms, optionally containing at least one hetero atom;
R₂ represents
• OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, a saturated or unsaturated C₁-C₁₀ alkyl radical, a saturated or unsaturated ring C³, which is separate or fused to another ring C⁴, optionally containing at least one hetero atom, the alkyl radicals and the rings also possibly being substituted with at least one substituent A₁ in which R₇ and R'₇, which may be identical or different, denote:
• a hydrogen atom or a linear or branched, saturated or unsaturated C₁-C₁₀ alkyl radical,
• an aromatic ring C₅ optionally including at least one hetero atom, optionally substituted with at least one substituent A₂; and
in which the hetero atoms are chosen from N, O and S and a combination thereof.

27. Composition according to one of Claims 19 to 26, **characterized in that** the salt of the compound of formula (I) or (III) is a salt chosen from the sodium and potassium salts, the zinc (Zn²⁺), calcium (Ca²⁺), copper (Cu²⁺), iron (Fe²⁺), strontium (Sr²⁺), magnesium (Mg²⁺), ammonium and manganese (Mn²⁺) salts, the triethanolamine, monoethanolamine, diethanolamine, hexadecylamine, N,N,N',N'-tetrakis(2-hydroxypropyl)ethylenediamine and tris(hydroxymethyl)aminomethane salts, and the hydroxides, carbonates, sulphates, phosphates, halides and nitrates.

28. Composition according to one of Claims 19 to 27, **characterized in that** the compound of formula (I) is chosen from:
Compound 1 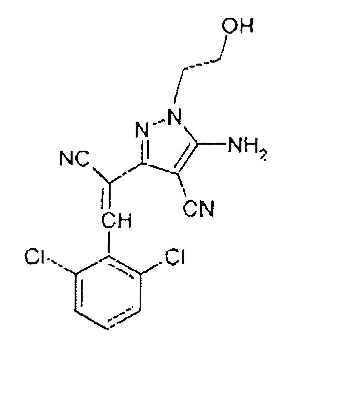
Compound 2 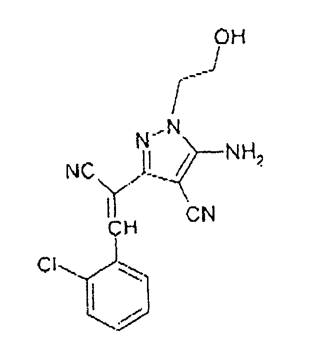
Compound 3 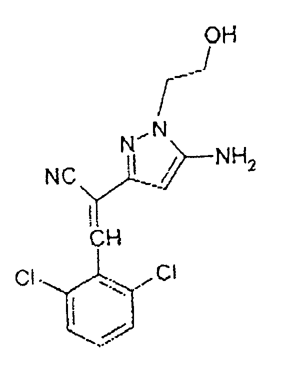
Compound 4 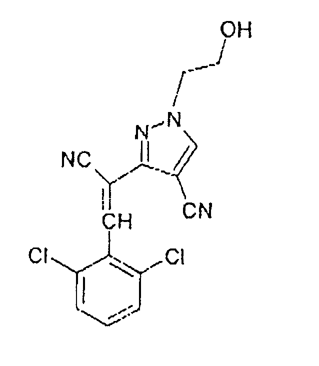
Compound 5 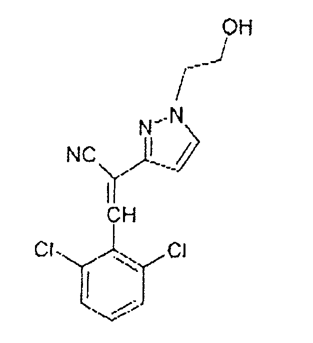
Compound 6 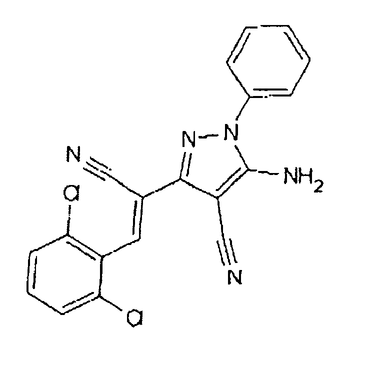
Compound 7 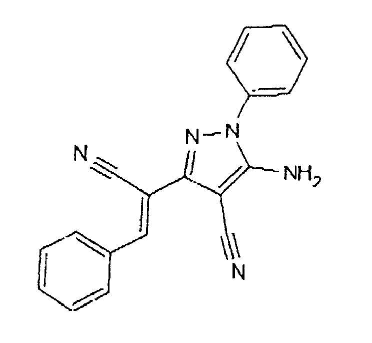
Compound 8 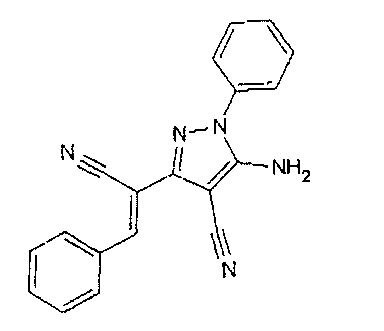
Compound 9 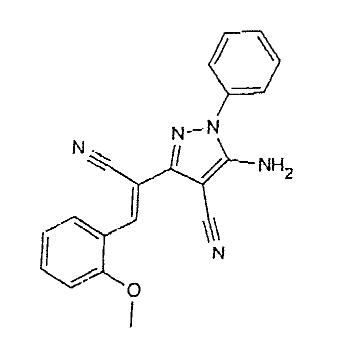
Compound 10 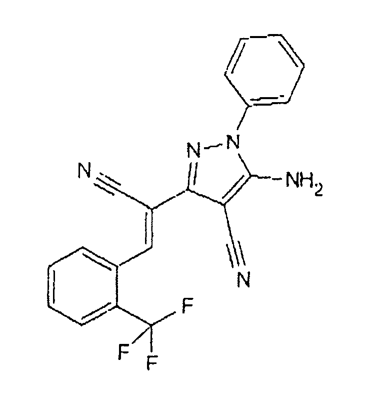
Compound 11

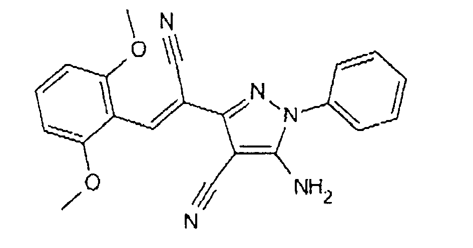

29. Composition according to one of Claims 19 to 28, **characterized in that** the compound of formula (I) or a mixture of compounds of formula (I) is used at a concentration ranging from 10⁻³% to 10% and preferably from 10⁻²% to 2% relative to the total weight of the composition.

30. Composition according to one of Claims 19 to 29, **characterized in that** it is in the form of a hair cream, a hair lotion, a shampoo, a conditioner or a mascara for the hair or the eyelashes.

31. Composition according to one of Claims 19 to 30, **characterized in that** it is in the form of an aqueous, alcoholic or aqueous-alcoholic solution or suspension.

32. Composition according to one of Claims 19 to 31, **characterized in that** it contains other ingredients chosen from solvents, aqueous-phase or oily-phase thickeners or gelling agents, dyestuffs that are soluble in the medium of the composition, fillers, pigments, antioxidants, preserving agents, fragrances, electrolytes, neutralizers, film-forming polymers, UV-blockers and cosmetic and pharmaceutical active agents, and mixtures thereof.

33. Composition according to one of Claims 19 to 32, **characterized in that** it also contains another active agent chosen from proteins, protein hydrolysates, amino acids, polyols, urea, allantoin, sugars and sugar derivatives, plant extracts, hydroxy acids, retinol derivatives, tocopherol derivatives, essential fatty acids, ceramides, essential oils, salicylic acid and its derivatives, for instance 5-n-octanoyl salicylic acid, hydroxy acid esters and phospholipids.

34. Composition according to one of Claims 19 to 33, **characterized in that** it contains at least one additional active compound that promotes the regrowth and/or limits the loss of keratin fibres.

35. Composition according to one of Claims 19 to 34, **characterized in that** it contains at least one additional active compound that promotes the regrowth and/or limits the loss of keratin fibres, chosen from aminexil, 6-O-[(9Z,12Z)octadeca-9,12-dienoyl]hexapyranose, lipoxygenase inhibitors, bradykinin inhibitors, prostaglandins and derivatives thereof, prostaglandin receptor agonists or antagonists, non-prostanoic prostaglandin analogues, vasodilators, antiandrogens, cyclosporins and analogues thereof, antimicrobial agents, anti-inflammatory agents, retinoids, benzalkonium chloride, benzethonium chloride, phenol, oestradiol, chlorpheniramine maleate, chlorophylline derivatives, cholesterol, cysteine, methionine, menthol, peppermint oil, calcium pantothenate, panthenol, resorcinol, protein kinase C activators, glycosidase inhibitors, glycosaminoglycanase inhibitors, pyroglutamic acid esters, hexosaccharidic or acylhexosaccharidic acids, aryl-substituted ethylenes, N-acyl amino acids, flavonoids, ascomycin derivatives and analogues, histamine antagonists, saponins, proteoglycanase inhibitors, oestrogen agonists and antagonists, pseudoterines, cytokines and growth factor promoters, IL-1 or IL-6 inhibitors, IL-10 promoters, TNF inhibitors, benzophenones, hydantoin, octopirox, retinoic acid, antipruriginous agents, antiparasitic agents, antifungal agents, nicotinic acid esters, calcium antagonists, hormones, triterpenes, antiandrogens, steroidal or non-steroidal 5-α-reductase inhibitors, potassium-channel agonists and FP receptor agonists, and mixtures thereof.

36. Composition according to Claim 35, **characterized in that** the additional compound is chosen from aminexil, FP receptor agonists and vasodilators.

37. Composition according to one of Claims 34 to 36, **characterized in that** the additional active compound is chosen from aminexil, minoxidil, latanoprost, butaprost and travoprost.

38. Cosmetic process for treating keratin fibres and/or the skin from which the said fibres emerge, **characterized in that** it consists in applying to the fibres and/or the skin a cosmetic composition as defined in any of Claims 19 to 37, leaving this composition in contact with the fibres and/or the skin, and optionally rinsing it out.

39. Cosmetic care and/or makeup process for human eyelashes, to improve their condition and/or appearance, **characterized in that** it consists in applying to the eyelashes and/or the eyelids a composition as defined in any one of Claims 19 to 37 in the form of a mascara, and leaving this composition in contact with the eyelashes and/or the eyelids.

40. Cosmetic care process for human hair and/or the scalp, to improve their condition and/or appearance, **characterized in that** it consists in applying to the hair and/or the scalp a cosmetic composition as defined in any one of Claims 19 to 37, leaving the composition in contact with the hair and/or the scalp, and optionally rinsing it out.

## Patentansprüche

1. Kosmetische Verwendung mindestens eines Styrylpyrazols der Formel (I) oder eines seiner Salze: 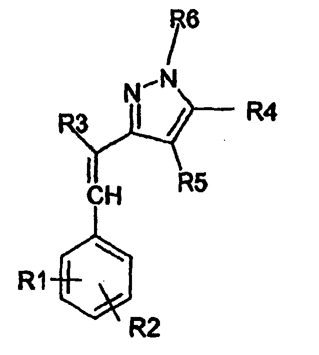 wobei in der Formel:
- die Gruppen R₁, R₂, R₄ und R₅, die gleich oder verschieden sind, ausgewählt sind unter Wasserstoff, Halogenen, den Gruppen OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, gesättigten oder ungesättigten, geradkettigen oder verzweigten C₁₋₂₀-Alkylgruppen, gesättigten oder ungesättigten Ringen mit 4 bis 7 Atomen, die gegebenenfalls mindestens ein Heteroatom enthalten, wobei diese Ringe isoliert oder verbunden sein können, wobei die Alkylgruppen und die Ringe ferner mit mindestens einem Substituenten A₁ substituiert sein können, wobei R₇, R'₇, R"₇ und R"'₇ unabhängig voneinander Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt und gegebenenfalls mit mindestens einem Substituenten A₂ substituiert sind;
- R₃ ausgewählt ist unter CN, COOR₈, CONR₈R'₈, COR₈, SO₂R₈, SO₂NR₈R'₈, wobei R₈ und R'₈ unabhängig voneinander Wasserstoff oder eine geradkettige oder verzweigte C₁₋₂₀-Alkylgruppe oder einen Ring mit 4 bis 7 Atomen bedeuten, der isoliert vorliegt oder mit einem anderen Ring verbunden ist und gegebenenfalls mindestens ein Heteroatom enthält, wobei die Alkylgruppe oder die Ringe gesättigt oder ungesättigt und gegebenenfalls mit mindestens einem Substituenten A₃ substituiert sind;
- R₆ ausgewählt ist unter Wasserstoff, den Gruppen COOR₉, COR₉, CSR₉, COSR₉, CONR₉R'₉, SO₂R₉, SO₂NR₉R'₉, geradkettigen oder verzweigten, gesättigten oder ungesättigten C₁₋₂₀-Alkylgruppen und gesättigten oder ungesättigten Ringen mit 4 bis 7 Atomen, die gegebenenfalls mindestens ein Heteroatom enthalten, wobei die Ringe isoliert vorliegen oder verbunden sein können, wobei die Alkylgruppen und Ringe ferner mit mindestens einem Substituenten A₄ substituiert sein können, wobei die Gruppen R₉ und R'₉, die gleich oder verschieden sind, Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt und gegebenenfalls mit mindestens einem Substituenten A₅ substituiert sind;
- wobei die Gruppen A₁, A₂, A₃, A₄ und A₅ unabhängig voneinander unter den Halogenen, den Gruppen OR₁₀, SRio, NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R'"₁₀, NR₁₀CSNR'₁₀R"₁₀, NR₁₀CSR'₁₀ ausgewählt sind, wobei Rio, R'₁₀, R"₁₀, R"'₁₀, die gleich oder verschieden sind, Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt vorliegen,
in einer kosmetischen Zusammensetzung für die Pflege und/oder zum Schminken von menschlichen Keratinfasern, um ihr Wachstum zu induzieren und/oder zu stimulieren, ihr Ausfallen zu verlangsamen und/oder ihre Dichte zu erhöhen.

2. Kosmetische Verwendung mindestens eines Styrylpyrazols der Formel (I) oder eines seiner Salze: 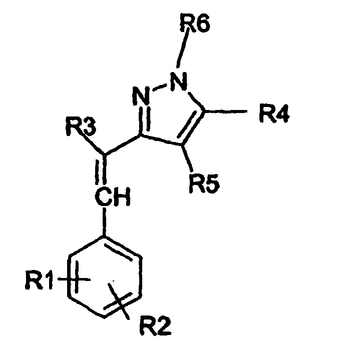 wobei in der Formel:
- die Gruppen R₁, R₂, R₄ und R₅, die gleich oder verschieden sind, ausgewählt sind unter Wasserstoff, Halogenen, den Gruppen OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R'"₇, NR₇CSR'₇, NR₇CSNR'₇R"₇, gesättigten oder ungesättigten, geradkettigen oder verzweigten C₁₋₂₀-Alkylgruppen, gesättigten oder ungesättigten Ringen mit 4 bis 7 Atomen, die gegebenenfalls mindestens ein Heteroatom enthalten, wobei diese Ringe isoliert oder verbunden sein können, wobei die Alkylgruppen und die Ringe ferner mit mindestens einem Substituenten A₁ substituiert sein können, wobei R₇, R'₇, R"₇ und R"'₇ unabhängig voneinander Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt und gegebenenfalls mit mindestens einem Substituenten A₂ substituiert sind;
- R₃ ausgewählt ist unter CN, COOR₈, CONR₈R'₈, COR₈, SO₂R₈, SO₂NR₈R₈'_{,} wobei R₈ und R'₈ unabhängig voneinander Wasserstoff oder eine geradkettige oder verzweigte C₁₋₂₀-Alkylgruppe oder einen Ring mit 4 bis 7 Atomen bedeuten, der isoliert vorliegt oder mit einem anderen Ring verbunden ist und gegebenenfalls mindestens ein Heteroatom enthält, wobei die Alkylgruppe oder die Ringe gesättigt oder ungesättigt und gegebenenfalls mit mindestens einem Substituenten A₃ substituiert sind;
- R₆ ausgewählt ist unter Wasserstoff, den Gruppen COOR₉, COR₉, CSR₉, COSR₉, CONR₉R'₉, SO₂R₉, SO₂NR₉R'₉, geradkettigen oder verzweigten, gesättigten oder ungesättigten C₁₋₂₀-Alkylgruppen und gesättigten oder ungesättigten Ringen mit 4 bis 7 Atomen, die gegebenenfalls mindestens ein Heteroatom enthalten, wobei die Ringe isoliert vorliegen oder verbunden sein können, wobei die Alkylgruppen und Ringe ferner mit mindestens einem Substituenten A₄ substituiert sein können, wobei die Gruppen R₉ und R'₉, die gleich oder verschieden sind, Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt und gegebenenfalls mit mindestens einem Substituenten A₅ substituiert sind;
- wobei die Gruppen A₁, A₂, A₃, A₄ und A₅ unabhängig voneinander unter den Halogenen, den Gruppen OR₁₀, SR₁₀, NR₁₀R'₁₀, COOR₁₀, CH₂COOR₁₀, CONR₁₀R'₁₀, CF₃, CN, NR₁₀COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R'"₁₀, NR₁₀CSNR'₁₀R"₁₀, NR₁₀CSR'₁₀ ausgewählt sind, wobei Rio, R'₁₀, R"₁₀, R'"₁₀, die gleich oder verschieden sind, Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt vorliegen,
als Inhibitor der 15-Hydroxy-prostaglandin-Dehydrogenase, insbesondere der menschlichen 15-Hydroxy-prostaglandin-Dehydrogenase.

3. Verwendung nach einem der vorhergehenden Ansprüche, **dadurch gekennzeichnet, dass** es sich bei den Keratinfasern um Haare, Augenbrauen, Wimpern, Vollbärte, Oberlippenbärte und Schamhaare handelt.

4. Verwendung mindestens eines Styrylpyrazols der Formel (I) oder eines seiner Salze: 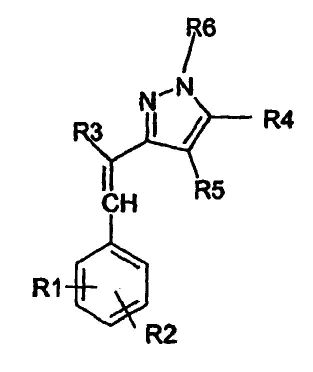 wobei in der Formel:
- die Gruppen R₁, R₂, R₄ und R₅, die gleich oder verschieden sind, ausgewählt sind unter Wasserstoff, Halogenen, den Gruppen OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, gesättigten oder ungesättigten, geradkettigen oder verzweigten C₁₋₂₀-Alkylgruppen, gesättigten oder ungesättigten Ringen mit 4 bis 7 Atomen, die gegebenenfalls mindestens ein Heteroatom enthalten, wobei diese Ringe isoliert oder verbunden sein können, wobei die Alkylgruppen und die Ringe ferner mit mindestens einem Substituenten A₁ substituiert sein können, wobei R₇, R'₇, R"₇ und R"'₇ unabhängig voneinander Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt und gegebenenfalls mit mindestens einem Substituenten A₂ substituiert sind;
- R₃ ausgewählt ist unter CN, COOR₈, CONR₈R'_{8,} COR₈, SO₂R₈, SO₂NR₈R'₈, wobei R₈ und R'₈ unabhängig voneinander Wasserstoff oder eine geradkettige oder verzweigte C₁₋₂₀-Alkylgruppe oder einen Ring mit 4 bis 7 Atomen bedeuten, der isoliert vorliegt oder mit einem anderen Ring verbunden ist und gegebenenfalls mindestens ein Heteroatom enthält, wobei die Alkylgruppe oder die Ringe gesättigt oder ungesättigt und gegebenenfalls mit mindestens einem Substituenten A₃ substituiert sind;
- R₆ ausgewählt ist unter Wasserstoff, den Gruppen COOR₉, COR₉, CSR₉, COSR₉, CONR₉R'₉, SO₂R₉, SO₂NR₉R'₉, geradkettigen oder verzweigten, gesättigten oder ungesättigten C₁₋₂₀-Alkylgruppen und gesättigten oder ungesättigten Ringen mit 4 bis 7 Atomen, die gegebenenfalls mindestens ein Heteroatom enthalten, wobei die Ringe isoliert vorliegen oder verbunden sein können, wobei die Alkylgruppen und Ringe ferner mit mindestens einem Substituenten A₄ substituiert sein können, wobei die Gruppen R₉ und R'₉, die gleich oder verschieden sind, Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt und gegebenenfalls mit mindestens einem Substituenten A₅ substituiert sind;
- wobei die Gruppen A₁, A₂, A₃, A₄ und A₅ unabhängig voneinander unter den Halogenen, den Gruppen OR₁₀, SR₁₀, NR₁₀R'₁₀, COOR₁₀, CH₂COOR₁₀, CONR₁₀R'₁₀, CF₃, CN, NR₁₀COR'₁₀, SO₂R₁₀, SO₂N₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R"'₁₀, NR₁₀CSNR'₁₀R"₁₀, NR₁₀CSR'₁₀ ausgewählt sind, wobei Rio, R'₁₀, R"₁₀, R'"₁₀, die gleich oder verschieden sind, Wasserstoff, eine geradkettige oder verzweigte C₁₋₂₀-Alkylgruppe oder einen Ring mit 4 bis 7 Atomen bedeuten, der gegebenenfalls mindestens ein Heteroatom enthält und isoliert vorliegt oder mit einem anderen Ring verbunden ist, wobei die Alkylgruppe oder die Ringe gesättigt oder ungesättigt vorliegen,
für die Herstellung einer Zusammensetzung für die Haarbehandlung beim Menschen, die für die Behandlung androgener Alopezie und/oder für die Behandlung natürlicher Alopezie vorgesehen ist.

5. Verwendung nach einem der vorhergehenden Ansprüche, **dadurch gekennzeichnet, dass** das Styrylpyrazol die folgende Formel (II) aufweist, oder eines seiner Salze: 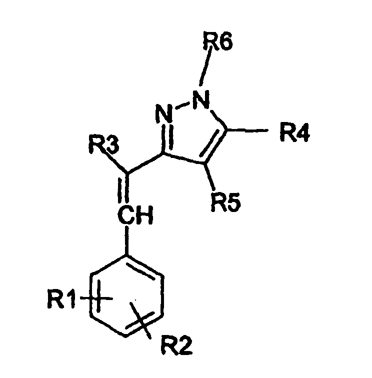 wobei in der Formel:
- die Gruppen R₁, R₂, R₄ und R₅ unabhängig voneinander H. Halogen, OR₇, SR₇, NR₇R'₇, COOR₇, CONR₇R'₇, CF₃, CN, eine geradkettige oder verzweigte C₁₋₁₀-Alkylgruppe, einen gesättigten oder ungesättigten Ring, der isoliert vorliegt oder mit einem anderen Ring verbunden ist und gegebenenfalls mindestens ein Heteroatom enthält, wobei die Alkylgruppen und Ringe ferner mit mindestens einem Substituenten A₁ substituiert sein können, wobei R₇ und R₇' unabhängig voneinander H, C₁-₁₀-Alkyl oder einen isolierten Ring oder mit einem anderen Ring verbundenen Ring bedeuten;
- R₃ CN, COOR₈, CONR₈R'₈, COR₈, wobei R₈ und R'₈ unabhängig voneinander H, C₁₋₁₀-Alkyl oder einen isolierten oder mit einem anderen Ring verbundenen Ring, der gegebenenfalls mindestens ein Heteroatom enthält, bedeuten, wobei die Ringe gesättigt oder ungesättigt sind und gegebenenfalls mit mindestens einem Substituenten A₁ substituiert sind;
- R₆ Wasserstoff, COOR₉, COR₉, eine gesättigte oder ungesättigte C₁₋₁₀-Alkylgruppe oder einen gesättigten oder ungesättigten Ring, der isoliert vorliegt oder mit einem anderen Ring verbunden ist und gegebenenfalls mindestens ein Heteroatom enthält, wobei die Alkylgruppen und Ringe ferner mit mindestens einem Substituenten A₁ substituiert sein können, wobei R₉ und R'₉ unabhängig voneinander H, C₁₋₂₀-Alkyl oder einen isolierten oder mit einem anderen Ring verbundenen Ring bedeuten;
- wobei die Ringe 5 bis 6 Atome enthalten,
- wobei die Heteroatome O, N, S oder deren Kombinationen bedeuten.

6. Verwendung nach einem der vorhergehenden Ansprüche, **dadurch gekennzeichnet, dass** mindestens eine der Gruppen R₁ und R₂ ein Wasserstoffatom, ein Halogenatom, OR₇ oder CF₃ bedeutet.

7. Verwendung nach einem der vorhergehenden Ansprüche, **dadurch gekennzeichnet, dass** sich R₁ und R₂ in ortho-Stellung zur Brücke zum Pyrazolteil an dem Phenylring befinden.

8. Verwendung nach einem der vorhergehenden Ansprüche, **dadurch gekennzeichnet, dass** R₁ und/oder R₂ ein Halogenatom und insbesondere ein Chloratom bedeuten.

9. Verwendung nach einem der vorhergehenden Ansprüche, **dadurch gekennzeichnet, dass** R₃ CN ist.

10. Verwendung nach dem vorhergehenden Anspruch, **dadurch gekennzeichnet, dass** R₄, R₅ und R₆ unabhängig voneinander NH₂, H, CN, eine gegebenenfalls mit OR₁₀ substituierte C₁₋₁₀-Alkylgruppe, einen gesättigten oder ungesättigten Kohlenwasserstoffring mit 5 oder 6 Atomen bedeuten.

11. Verwendung nach einem der vorhergehenden Ansprüche, **dadurch gekennzeichnet, dass** R₆CH₂CH₂OH oder eine Phenylgruppe bedeutet.

12. Verwendung nach einem der vorhergehenden Ansprüche, **dadurch gekennzeichnet, dass** R₄ NH₂ oder H bedeutet.

13. Verwendung nach einem der vorhergehenden Ansprüche, **dadurch gekennzeichnet, dass** R₅ CN oder H bedeutet.

14. Verwendung nach einem der vorhergehenden Ansprüche, **dadurch gekennzeichnet, dass** das Styrylpyrazol die folgende Formel (III) aufweist, oder eines seiner Salze: 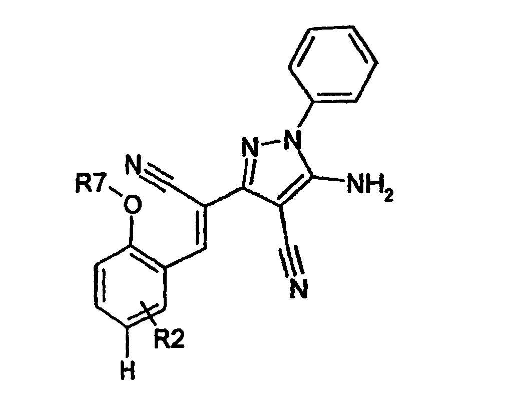 R₇ bedeutet:
a) eine geradkettige oder verzweigte, gesättigte oder ungesättigte, gegebenenfalls mit mindestens einem Substituenten A₁ substituierte C₁₋₁₀-Alkylgruppe; oder
b) einen gesättigten oder ungesättigten Ring C¹ mit 4 bis 7 Atomen, der gegebenenfalls mindestens ein Heteroatom enthält und/oder gegebenenfalls mit mindestens einem Substituenten A₁ substituiert ist und/oder gegebenenfalls mit mindestens einem gesättigten oder ungesättigten Ring C² mit 4 bis 7 Atomen verbunden ist, der gegebenenfalls mindestens ein Heteroatom enthält;
R₂ bedeutet
· OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, eine gesättigte oder ungesättigte C₁₋₁₀-Alkylgruppe, einen gesättigten oder ungesättigten Ring C³, der isoliert vorliegt oder mit einem weiteren Ring C⁴ verbunden ist, der gegebenenfalls mindestens ein Heteroatom enthält, wobei die Alkylgruppen und die Ringe ferner mit mindestens einem Substituenten A₁ substituiert sein können, wobei die Gruppen R₇ und R'₇ gleich oder verschieden sind und bedeuten:
· Wasserstoff oder eine geradkettige oder verzweigte, gesättigte oder ungesättigte C₁-₁₀-Alkylgruppe,
· einen aromatischen Ring C₅, der gegebenenfalls mindestens ein Heteroatom enthält und gegebenenfalls mit mindestens einem Substituenten A₂ substituiert ist;
wobei die Heteroatome unter N, O, S und deren Kombinationen ausgewählt sind.

15. Verwendung nach einem der vorhergehenden Ansprüche, **dadurch gekennzeichnet, dass** das Salz der Verbindung (I) ein Salz ist, das unter den Natriumsalzen, Kaliumsalzen, Zinksalzen (Zn²⁺), Calciumsalzen (Ca²⁺), Kupfersalzen (Cu²⁺), Eisensalzen (Fe²⁺), Strontiumsalzen (Sr²⁺), Magnesiumsalzen (Mg²⁺), Ammoniumsalzen, Mangansalzen (Mn²⁺), Triethanolaminsalzen, Monoethanolaminsalzen, Diethanolaminsalzen, Hexadecylaminsalzen, N,N,N',N'-Tetrakis-(2-hydroxypropyl)-ethylendiaminsalzen, Tris-hydroxymethylaminomethansalzen, Hydroxiden, Carbonaten, Sulfaten, Phosphaten, Halogeniden und Nitraten ausgewählt ist.

16. Verwendung nach einem der vorhergehenden Ansprüche, **dadurch gekennzeichnet, dass** die Verbindung der Formel (I) ausgewählt ist unter:

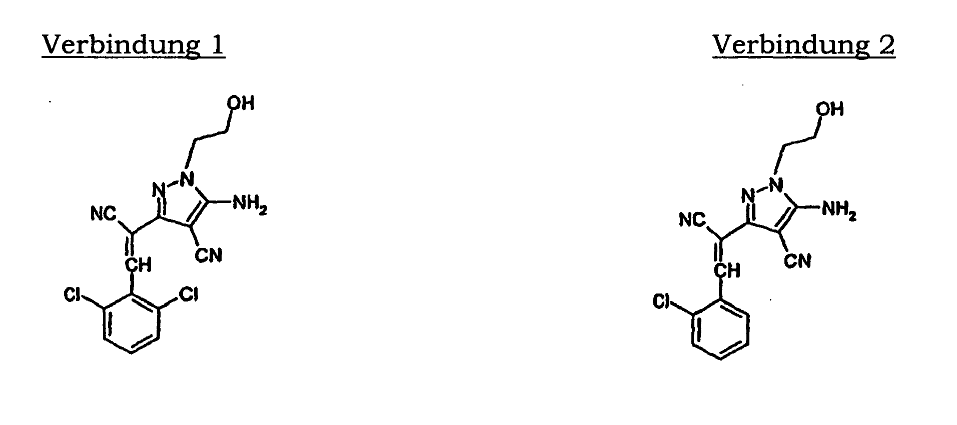

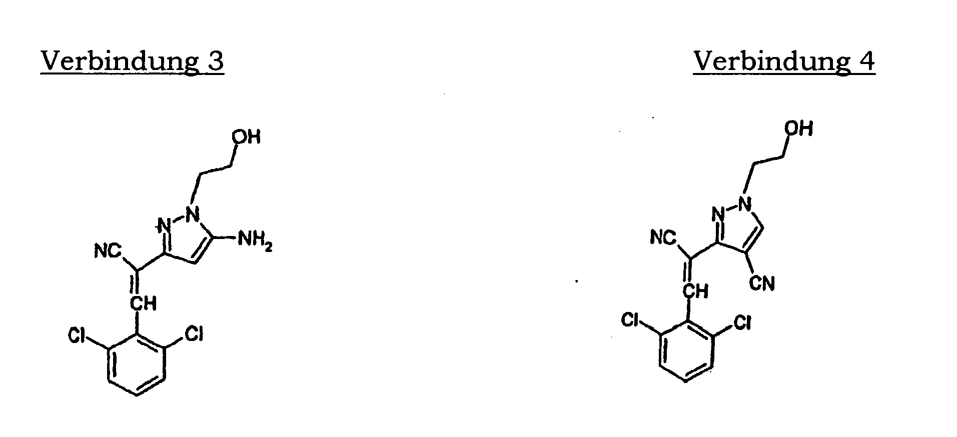

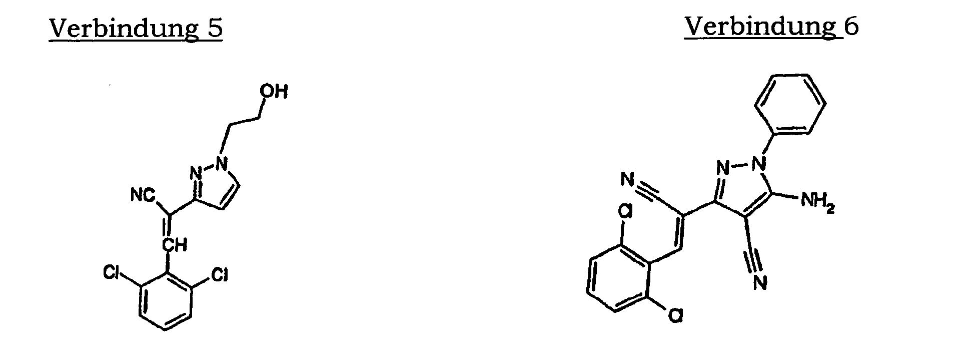

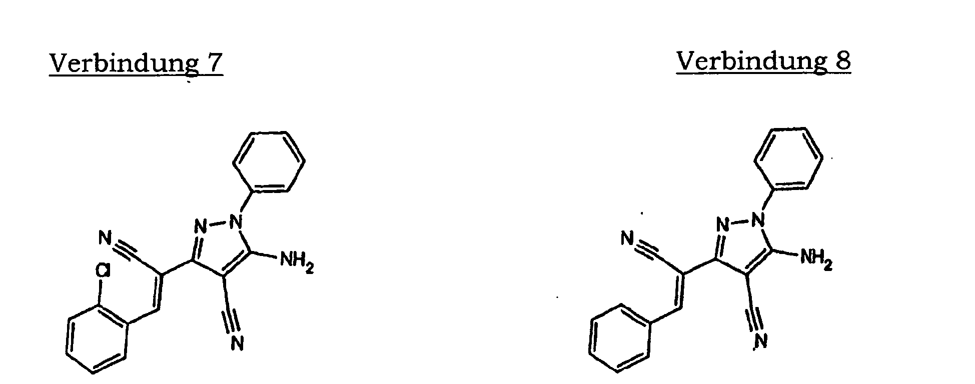

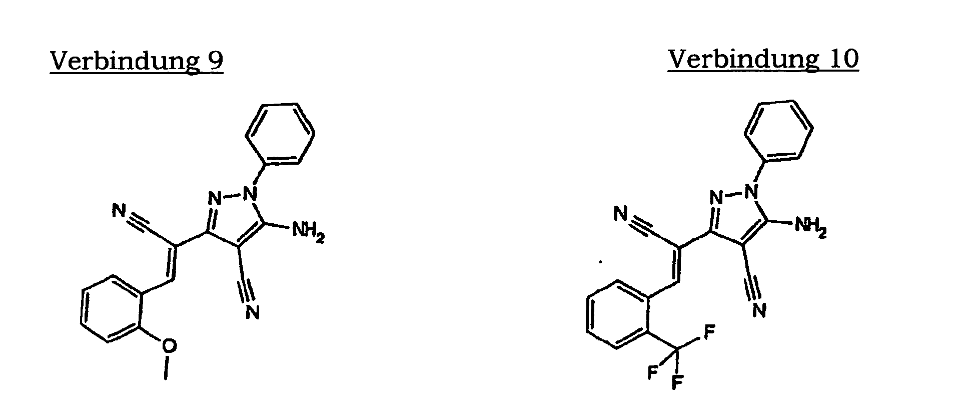

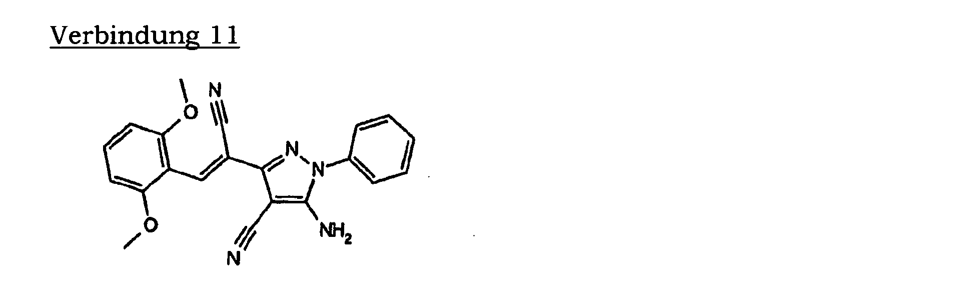

17. Verwendung nach einem der vorhergehenden Ansprüche, **dadurch gekennzeichnet, dass** die Verbindung der Formel (I) oder ein Gemisch aus Verbindungen der Formel (I) in einer Konzentration von 10⁻³ bis 10 % und vorzugsweise 10⁻² bis 2 %, bezogen auf das Gesamtgewicht der Zusammensetzung, verwendet wird.

18. Verwendung nach einem der vorhergehenden Ansprüche, **dadurch gekennzeichnet, dass** die Zusammensetzung eine Zusammensetzung für die topische Anwendung ist.

19. Zusammensetzung für die Haarpflege oder zum Schminken von Keratinsubstanzen, die ein physiologisch akzeptables Medium und in einer wirksamen Menge mindestens ein Styrylpyrazol der Formel (I) oder eines seiner Salze nach einem der Ansprüche 1 bis 16 enthält, wobei sich R₁ und R₂ an dem Phenylring in ortho-Stellung zur Brücke zum Pyrazolteil befinden.

20. Zusammensetzung nach Anspruch 19, **dadurch gekennzeichnet, dass** R₁ und/oder R₂ ein Halogenatom und insbesondere ein Chloratom bedeuten.

21. Zusammensetzung nach einem der Ansprüche 19 und 20, **dadurch gekennzeichnet, dass** R₃ CN bedeutet.

22. Zusammensetzung nach einem der Ansprüche 19 bis 21, **dadurch gekennzeichnet, dass** die Gruppen R₄, R₅ und R₆ unabhängig voneinander NH₂, H, CN, eine gegebenenfalls mit OR₁₀ substituierte C₁₋₁₀-Alkylgruppe, einen gesättigten oder ungesättigten Kohlenwasserstoffring mit 5 oder 6 Atomen bedeuten.

23. Zusammensetzung nach einem der Ansprüche 19 bis 22, **dadurch gekennzeichnet, dass** R₆ CH₂CH₂OH oder Phenyl bedeutet.

24. Zusammensetzung nach einem der Ansprüche 19 bis 23, **dadurch gekennzeichnet, dass** R₄ NH₂ oder H bedeutet.

25. Zusammensetzung nach einem der Ansprüche 19 bis 24, **dadurch gekennzeichnet, dass** R₅ CN oder H bedeutet.

26. Zusammensetzung nach einem der Ansprüche 19 bis 25, die ein physiologisch akzeptables Medium und in einer wirksamen Menge mindestens ein Styrylpyrazol der folgenden Formel (III) oder eines seiner Salze enthält: 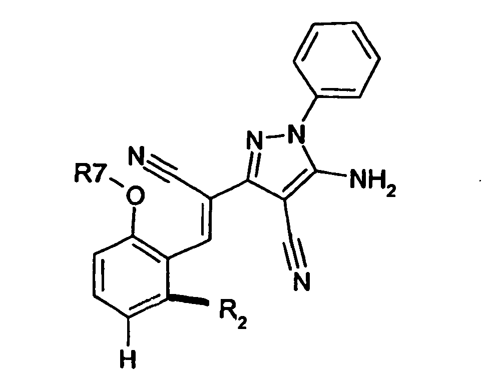 R₇ bedeutet:
a) eine geradkettige oder verzweigte, gesättigte oder ungesättigte, gegebenenfalls mit mindestens einem Substituenten A₁ substituierte C₁₋₁₀-Alkylgruppe; oder
b) einen gesättigten oder ungesättigten Ring C¹ mit 4 bis 7 Atomen, der gegebenenfalls mindestens ein Heteroatom enthält und/oder gegebenenfalls mit mindestens einem Substituenten A₁ substituiert ist und/oder gegebenenfalls mit mindestens einem gesättigten oder ungesättigten Ring C² mit 4 bis 7 Atomen verbunden ist, der gegebenenfalls mindestens ein Heteroatom enthält;
R₂ bedeutet:
· OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, eine gesättigte oder ungesättigte C₁₋₁₀-Alkylgruppe, einen gesättigten oder ungesättigten Ring C³, der isoliert vorliegt oder mit einem weiteren Ring C⁴ verbunden ist, der gegebenenfalls mindestens ein Heteroatom enthält, wobei die Alkylgruppen und die Ringe-ferner mit mindestens einem Substituenten A₁ substituiert sein können, wobei die Gruppen R₇ und R'₇ gleich oder verschieden sind und bedeuten:
· Wasserstoff oder eine geradkettige oder verzweigte, gesättigte oder ungesättigte C₁₋₁₀-Alkylgruppe,
· einen aromatischen Ring C₅, der gegebenenfalls mindestens ein Heteroatom enthält und gegebenenfalls mit mindestens einem Substituenten A₂ substituiert ist;
wobei die Heteroatome unter N, O, S und deren Kombinationen ausgewählt sind.

27. Zusammensetzung nach einem der Ansprüche 19 bis 26, **dadurch gekennzeichnet, dass** das Salz der Verbindung (I) oder (III) ein Salz ist, das unter den Natriumsalzen, Kaliumsalzen, Zinksalzen (Zn²⁺), Calciumsalzen (Ca²⁺), Kupfersalzen (Cu²⁺), Eisensalzen (Fe²⁺), Strontiumsalzen (Sr²⁺), Magnesiumsalzen (Mg²⁺), Mangansalzen (Mn²+), Ammoniumsalzen, Triethanolaminsalzen, Monoethanolaminsalzen, Diethanolaminsalzen, Hexadecylaminsalzen, N,N,N',N'-Tetrakis-(2-hydroxypropyl)-ethylendiamin-salzen, Tris-hydroxymethylaminomethansalzen, Hydroxiden, Carbonaten, Sulfaten, Phosphaten, Halogeniden und Nitraten ausgewählt ist.

28. Zusammensetzung nach einem der Ansprüche 19 bis 27, **dadurch gekennzeichnet, dass** die Verbindung der Formel (I) ausgewählt ist unter:

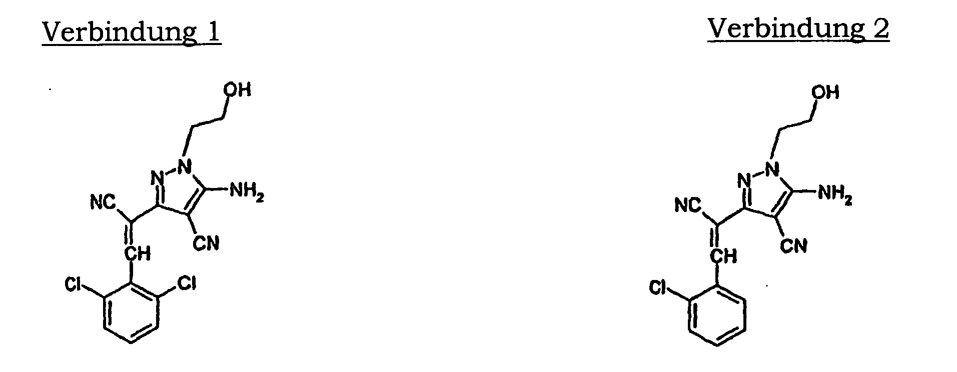

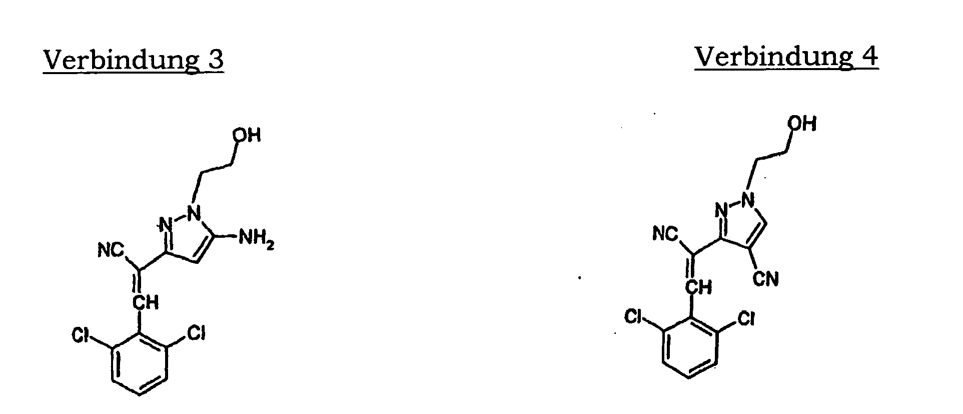

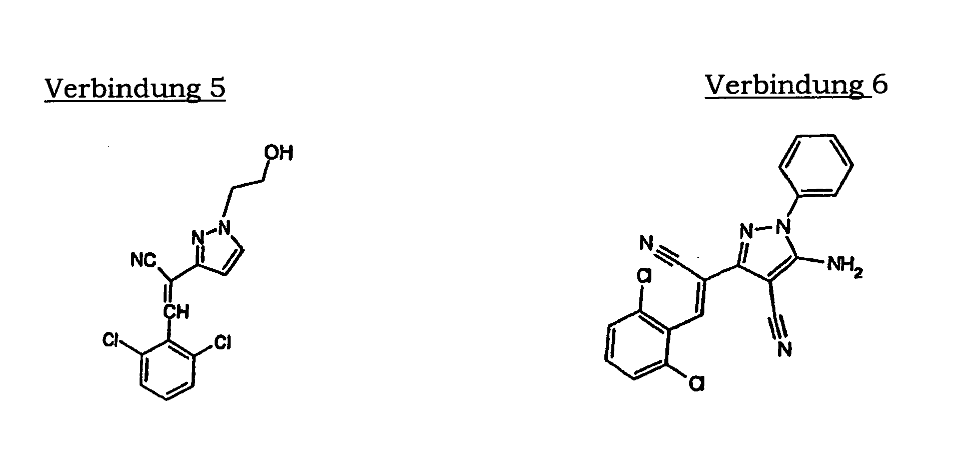

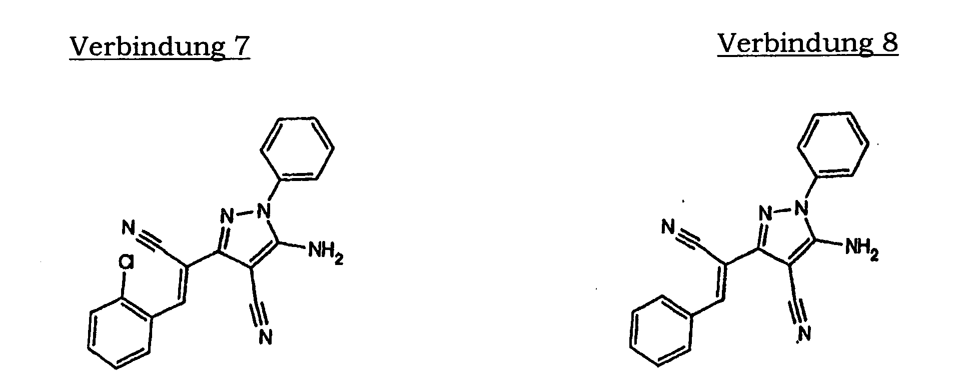

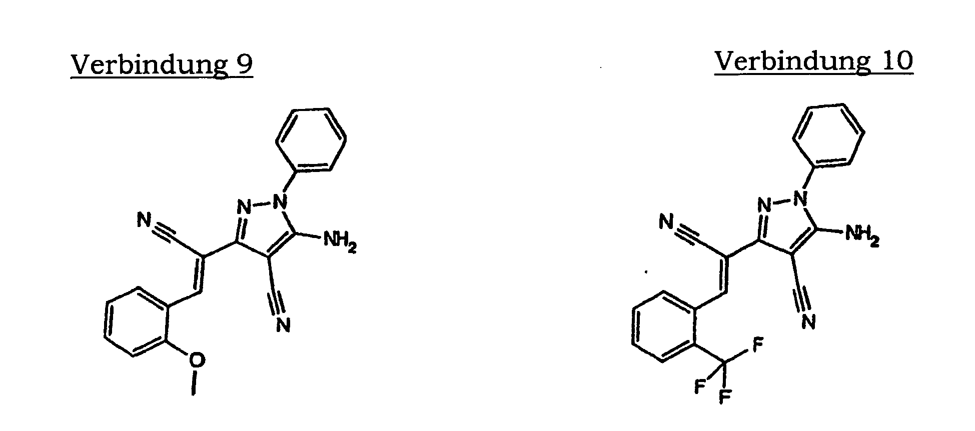

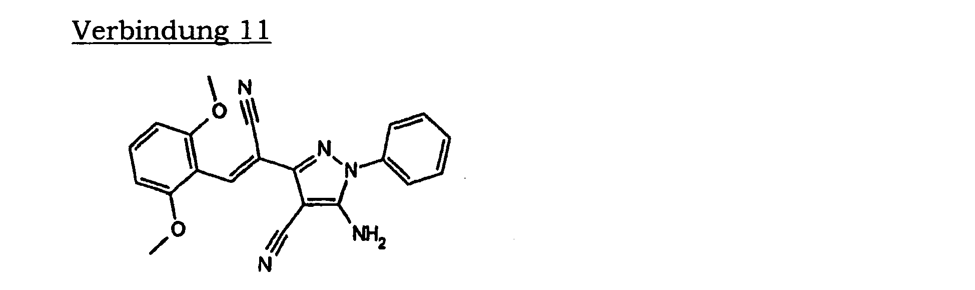

29. Zusammensetzung nach einem der Ansprüche 19 bis 28, **dadurch gekennzeichnet, dass** die Verbindung der Formel (I) oder ein Gemisch aus Verbindungen der Formel (I) in einer Konzentration von 10⁻³ bis 10 % und vorzugsweise 10⁻² bis 2 %, bezogen auf das Gesamtgewicht der Zusammensetzung, verwendet wird.

30. Zusammensetzung nach einem der Ansprüche 19 bis 29, **dadurch gekennzeichnet, dass** sie als Creme oder Lotion für die Haarbehandlung, Haarwaschmittel, Produkt, das nach der Haarwäsche angewandt wird, Mascara für das Haar oder die Wimpern vorliegt.

31. Zusammensetzung nach einem der Ansprüche 19 bis 30, **dadurch gekennzeichnet, dass** sie als wässrige, alkoholische oder wässrig-alkoholische Lösung oder Suspension vorliegt.

32. Zusammensetzung nach einem der Ansprüche 19 bis 31, **dadurch gekennzeichnet, dass** sie weitere Bestandteile enthält, die unter den Lösungsmitteln, Verdickungsmitteln oder Gelbildnern für die wässrige Phase oder Ölphase, in dem Medium der Zusammensetzung löslichen Farbstoffen, Füllstoffen, Pigmenten, Antioxidantien, Konservierungsmitteln, Parfums, Elektrolyten, Neutralisationsmitteln, filmbildenden Polymeren, UV-Blockern, kosmetischen und pharmazeutischen Wirkstoffen und deren Gemischen ausgewählt sind.

33. Zusammensetzung nach einem der Ansprüche 19 bis 32, **dadurch gekennzeichnet, dass** sie ferner mindestens einen Wirkstoff enthält, der unter den Proteinen, Proteinhydrolysaten, Aminosäuren, Polyolen, Harnstoff, Allantoin, Zuckern und Zuckerderivaten, Pflanzenextrakten, Hydroxysäuren, Retinolderivaten oder Tocopherolderivaten, essentiellen Fettsäuren, Ceramiden, etherischen Ölen, Salicylsäure und ihren Derivaten, wie 5-*n*-Octanoylsalicylsäure, Estern von Hydroxysäuren und Phospholipiden ausgewählt ist.

34. Zusammensetzung nach einem der Ansprüche 19 bis 33, **dadurch gekennzeichnet, dass** sie mindestens einen ergänzenden Wirkstoff enthält, der das Haarwachstum fördert und/oder das Ausfallen von Keratinfasern begrenzt.

35. Zusammensetzung nach einem der Ansprüche 19 bis 34, **dadurch gekennzeichnet, dass** sie mindestens einen ergänzenden Wirkstoff enthält, der das Haarwachstum fördert und/oder das Ausfallen von Keratinfasern begrenzt und der unter Aminexil, 6-O-[(9Z, 12Z)-Octadeca-9,12-dienoyl]-hexapyranose, Inhibitoren der Lipoxygenase, Inhibitoren von Bradykinin, Prostaglandinen und ihren Derivaten, Agonisten oder Antagonisten von Prostaglandin-Rezeptoren, nichtprostanoischen Analoga von Prostaglandinen, Vasodilatatoren, Antiandrogenen, Cyclosporinen und ihren Analoga, antimikrobiellen Wirkstoffen, entzündungshemmenden Wirkstoffen, Retinoiden, Benzalkoniumchlorid, Benzethoniumchlorid, Phenol, Estradiol, Chlorpheniraminmaleat, Chlorophyllinderivaten, Cholesterin, Cystein, Methionin, Menthol, Pfefferminzöl, Calciumpanthotenat, Panthenol, Resorcin, Aktivatoren der Proteinkinase C, Glycosidase-Inhibitoren, Glycosaminoglycanase-Inhibitoren, Pyroglutaminsäureestern, Hexosaccharidsäuren oder Acylhexosaccharinsäuren, arylsubstituierten Ethylenen, N-acylierten Aminosäuren, Flavonoiden, Derivaten und Analoga von Ascomycin, Histamin-Antagonisten, Saponinen, Proteoglycanase-Inhibitoren, Agonisten und Antagonisten von Östrogenen, Pseudoterinen, Cytokinen und Promotoren von Wachstumsfaktoren, Inhibitoren von IL-1 oder IL-6, Promotoren von IL-10, TNF-Inhibitoren, Benzophenonen, Hydantoin, Octopirox, Retinsäure, juckreizstillenden Wirkstoffen, antiparasitären Wirkstoffen, antimykotischen Wirkstoffen, Nicotinsäureestem, Calcium-Antagonisten, Hormonen, Triterpenen, antiandrogenen Wirkstoffen, steroidalen oder nichtsteoridalen Inhibitoren von 5-α-Reduktasen, Kaliumkanal-Agonisten, Agonisten des FP-Rezeptors und deren Gemischen ausgewählt ist.

36. Zusammensetzung nach Anspruch 35, **dadurch gekennzeichnet, dass** die ergänzende Verbindung unter Aminexil, Agonisten des FP-Rezeptors und Vasodilatatoren ausgewählt ist.

37. Zusammensetzung nach einem der Ansprüche 34 bis 36, **dadurch gekennzeichnet, dass** der zusätzliche Wirkstoff unter Aminexil, Minoxidil, Latanoprost, Butaprost und Travoprost ausgewählt ist.

38. Verfahren zur kosmetischen Behandlung von Keratinfasern und/oder Haut, aus der Keratinfasern hervorkommen, **dadurch gekennzeichnet, dass** es darin besteht, auf die Fasern und/oder die Haut eine kosmetische Zusammensetzung nach einem der Ansprüche 19 bis 37 aufzutragen, diese mit den Fasern und/oder der Haut in Kontakt zu belassen und gegebenenfalls zu spülen.

39. Verfahren für die kosmetische Pflege und/oder zum Schminken von menschlichen Wimpern, um ihren Zustand und/oder ihr Aussehen zu verbessern, **dadurch gekennzeichnet, dass** es darin besteht, auf die Wimpern und/oder die Lider eine Zusammensetzung nach einem der Ansprüche 19 bis 37 in Form von Mascara aufzubringen und diese in Kontakt mit den Wimpern und/oder den Lidern zu belassen.

40. Verfahren zur kosmetischen Pflege der Haare und/oder der menschlichen Kopfhaut, um ihren Zustand und/oder ihr Aussehen zu verbessern, **dadurch gekennzeichnet, dass** es darin besteht, auf die Haare und/oder die Kopfhaut eine kosmetische Zusammensetzung nach einem der Ansprüche 19 bis 37 aufzubringen und diese mit den Haaren und/oder der Kopfhaut in Kontakt zu belassen und gegebenenfalls zu spülen.
